# EUROPEAN PATENT SPECIFICATION

(11) **EP 3 945 290 B1**
(45) Date of publication and mention of the grant of the patent: **24.12.2025**
(21) Application number: 21185680.2
(22) Date of filing: 14.07.2021
(51) Int. Cl.: G01D 3/036, G01N 33/00

(54) **APPARATUSES, COMPUTER-IMPLEMENTED METHODS, AND COMPUTER PROGRAM PRODUCTS FOR DYNAMIC ITERATIVE BASELINE ADJUSTMENT**
COMPUTERIMPLEMENTIERTES VERFAHREN, VORRICHTUNG UND COMPUTERPROGRAMMPRODUKT ZUR DYNAMISCHEN ITERATIVEN BASISLINIENEINSTELLUNG
PROCÉDÉ MIS EN OEUVRE PAR ORDINATEUR, APPAREIL ET PRODUIT DE PROGRAMME INFORMATIQUE POUR UN AJUSTEMENT DYNAMIQUE ITÉRATIF DU SIGNAL DE BASE

(30) Priority: 31.07.2020 CN 202010759666
(43) Date of publication of application: 02.02.2022
(73) Proprietor: Rae Systems, Inc., Sunnyvale, CA 94089 (US)
(72) Inventor: ZHOU, Yang, Charlotte, 28202 (US); YE, Yifan, Charlotte, 28202 (US); XIE, Jiafu, Charlotte, 28202 (US); WEI, Na, Charlotte, 28202 (US); LIU, Ling, Charlotte, 28202 (US); SUN, Jiangbo, Charlotte, 28202 (US); LV, Hongling, Charlotte, 28202 (US); LI, Wenjuan, Charlotte, 28202 (US)
(74) Representative: Haseltine Lake Kempner LLP

(56) References cited:
- US-A1- 2019 033 274

## Description

### TECHNOLOGICAL FIELD

The present invention generally relates to calibration of baseline values associated with one or more sensor(s), and specifically to improved methodologies for baseline value adjustment utilizing dynamic iterative baseline adjustments in sensors to account for baseline inaccuracies, such as baseline drift.

### BACKGROUND

During the operational lifetime of a sensor, such as a PPB-level gas sensor, the baseline of the sensor may become inaccurate. For example, some gas sensors experience baseline drift as the sensor ages with time, and/or otherwise experiences changes due to changes in the ambient environment surrounding the sensor (e.g., changes in the ambient humidity and/or temperature). These baseline inaccuracies often introduce significant errors in the performance of the sensor even after the sensor undergoes conventional sensitivity compensation and/or baseline calibration. Applicant has discovered problems with current implementations attempting to current baseline inaccuracies. Through applied effort, ingenuity, and innovation, Applicant has solved many of these identified problems by developing embodied in the present invention, which are described in detail below.

US 2019/033274 A1 discloses a method of sensing including obtaining first sensor data points by a sensor, obtaining first reference data points, and determining a correlation between the first sensor data points and the first reference data points. The method of sensing further includes measuring second sensor data points by the sensor, obtaining second reference data points, and adjusting the second sensor data points using the correlation and the second reference data points to obtain corrected sensor data points. The method of sensing also includes determining sensed values from the corrected sensor data points and storing the sensed values.

### BRIEF SUMMARY

The present invention is defined by the appended claims. In general, embodiments of the present invention provide for improvements in overcoming errors in baseline value(s) of one or more sensors, such as gas sensors, utilizing one or more dynamic baseline adjustment iterative algorithm(s). Other implementations for dynamic baseline adjustment iterative algorithm(s) will be, or will become, apparent to one with skill in the art upon examination of the following figures and detailed description.

In general, embodiments of the present invention provided herein provide for improvements in accounting for baseline drift in sensors, such as gas sensors, utilizing one or more dynamic baseline adjustment iterative algorithm(s). In this regard, one or more of the embodiments described herein address problems associated with tracking and adapting to overcome baseline drift, so as to avoid inaccurate measurements resulting from baseline drift of a sensor or other inaccuracies between the true value of a baseline value and a current value of the baseline value being used. Other implementations for dynamic baseline adjustment iterative algorithm(s) will be, or will become, apparent to one with skill in the art upon examination of the following figures and detailed description.

In accordance with an aspect of the invention, a computer-implemented method of calibration of baseline values of a gas sensor is provided. The computer-implemented method is performable by any of a myriad of devices, apparatuses, systems, and/or components embodied in hardware, software, firmware, and/or a combination thereof as described herein. The computer-implemented method of the present invention comprises determining, from a set of measured values, a number of low-point measured values that exceeds a baseline updating threshold, where each measured value in the set of measured values is associated with a temperature interval of a set of temperature intervals, where each temperature interval of the set of temperature intervals is associated with a baseline value of a baseline value set, and where the set of measured values comprises a subset of low-point measured values comprising each measured value that is lower than the baseline value for the temperature interval corresponding to the measured value. The computer-implemented method further comprises determining an updated baseline value set comprising an updated baseline value for each temperature interval of the set of temperature intervals, where the updated baseline value for each temperature interval of the set of temperature intervals is determined based on at least one low-point measured value of the subset of low-point measured values that is associated with the temperature interval. The computer-implemented method further comprises updating the baseline value set to the updated baseline value set by, for each temperature interval of the set of temperature intervals, updating the baseline value of the baseline value set associated with the temperature interval to the updated baseline value associated with the temperature interval. The computer-implemented method further comprises performing a corrective baseline algorithm on the updated baseline value set. The computer-implemented method further comprises, for at least one baseline factor interval of the set of baseline factor intervals, assigning a factory default baseline as the baseline value for the baseline factor interval; and for at least one baseline factor interval of the set of baseline factor intervals, assigning the baseline value based on a linear interpolation between a first baseline value for a first baseline factor interval and a second baseline value for a second baseline factor interval, wherein the first baseline factor interval is an adjacent lower baseline factor interval and the second baseline factor interval is an adjacent higher baseline factor interval.

Additionally or alternatively, in some such example embodiments of the computer-implemented method, performing the corrective baseline algorithm comprises determining a monotonic trend of the updated baseline value set based on a change between each updated baseline value of the updated baseline value set for a change between each temperature interval in the set of temperature intervals.

Additionally or alternatively, in some such example embodiments of the computer-implemented method, the computer-implemented method further comprises determining, based on the set of measured values, a number of measured values that fall below a lower sensor limit; and setting the baseline updating threshold based on the number of measured values that fall below the lower sensor limit.

Additionally or alternatively, in some such example embodiments of the computer-implemented method, the computer-implemented method further comprises setting the baseline value set by: segmenting a temperature range into the set of temperature intervals based on an temperature interval size.

Additionally or alternatively, in some such example embodiments of the computer-implemented method, determining the set of measured values comprises the number of measured values that exceeds the baseline updating threshold comprises for each temperature interval of the set of temperature intervals: identifying the baseline value associated with the temperature interval; determining, from the set of measured values, a subset of measured values associated with the temperature interval; and for each measured value in the subset of measured values associated with the temperature interval: determining whether the measured value is lower than the baseline value; and in a circumstance where the measured value is lower than the baseline value, incrementing a count representing the number of low-point measured values.

Additionally or alternatively, in some such example embodiments of the computer-implemented method, the example computer-implemented method further comprise determining a set of abnormal measured values from the subset of low-point measured values; and generating an altered subset of low-point measured values by removing the set of abnormal measured values from the subset of low-point measured values, where the updated baseline value set is determined based on the altered subset of low-point measured values.

Additionally or alternatively, in some such example embodiments of the computer-implemented method, determining the updated baseline value set comprises for each temperature interval in the set of temperature intervals: determining an average low-point measured value for the temperature interval by averaging the subset of low-point measured values associated with the temperature interval; and setting the updated baseline value for the temperature interval to the average-low point measured value for the temperature interval.

Additionally or alternatively, in some such example embodiments of the computer-implemented method, the example computer-implemented method further comprises measuring a raw data value associated with a current operational temperature interval; and generating a corrected data value based on (1) the raw data value and (2) an updated baseline value associated with the current operational temperature interval.

Additionally or alternatively, in some such example embodiments of the computer-implemented method, the set of measured values comprises historical measured values received from a central server or plurality of connected devices.

In accordance with yet another aspect of the present invention, an apparatus is provided. The apparatus of the present invention comprises at least one processor and at least one memory, the at least one memory having computer-coded instructions stored thereon. The computer-coded instructions in execution with the at least one processor configure the apparatus for performing the operations of the aforementioned computer-implemented method.

In accordance with yet another aspect of the present invention, at least one computer program product is provided. The computer program product of the present invention comprises at least one non-transitory computer-readable storage medium having computer program code stored thereon. The computer program code in execution with a processor is configured for executing the operations of the aforementioned computer-implemented method.

### BRIEF DESCRIPTION OF THE DRAWINGS

Having thus described the embodiments of the invention in general terms, reference now will be made to the accompanying drawings, which are not necessarily drawn to scale, and wherein:
FIG. 1 illustrates a block diagram of a system that may be specially configured within which embodiments of the present invention may operate;
FIG. 2 illustrates a block diagram of an example apparatus that may be specially configured in accordance with an example embodiment of the present invention;
FIG. 3 illustrates a flowchart depicting operations of an example process for dynamic iterative baseline adjustment, in accordance with at least one embodiment of the present invention;
FIG. 4 illustrates another flowchart depicting operations of another example process for dynamic iterative baseline adjustment, in accordance with at least one embodiment of the present invention;
FIG. 5 illustrates another flowchart depicting additional operations of another example process for dynamic iterative baseline adjustment, in accordance with at least one embodiment of the present invention;
FIG. 6 illustrates another flowchart depicting additional operations of another example process for dynamic iterative baseline adjustment, in accordance with at least one embodiment of the present invention;
FIG. 7 illustrates another flowchart depicting additional operations of another example process for dynamic iterative baseline adjustment, in accordance with at least one embodiment of the present invention;
FIG. 8 illustrates another flowchart depicting additional operations of another example process for dynamic iterative baseline adjustment, in accordance with at least one embodiment of the present invention; and
FIG. 9 illustrates another flowchart depicting additional operations of another example process for dynamic iterative baseline adjustment, in accordance with at least one embodiment of the present invention.

### DETAILED DESCRIPTION

Embodiments of the present invention now will be described more fully hereinafter with reference to the accompanying drawings, in which some, but not all, embodiments of the invention are shown. Indeed, embodiments of the invention may be embodied in many different forms. Like numbers refer to like elements throughout.

### Overview

The use of sample collectors that collect and process samples from an environment relies on measurements from said sample collectors being sufficiently accurate. In one such example context, gas sensors are utilized for various purposes in environment sample collection and processing, for example atmospheric environment monitoring, odor monitoring, toxic gas monitoring, and/or the like. Such gas sensors monitor any of a myriad of pollutants, including without limitation CO, SO2, NO2, O3, VOC, and/or the like. At present, widespread monitoring networks are largely based on optical detection methods, which are highly accurate but are also high in cost thus making it difficult to further continue widespread, extensive use of such gas sensors throughout all areas (e.g., at a national and/or prefecture level) for several entities due to cost restrictions.

Alternative implementations utilize less costly gas sensors that function with lower accuracy at a lower cost. For example, some entities have begun to deploy air pollution monitoring micro-stations that utilize PPB-level electrochemical gas sensors as pollution monitoring methods, instead of high-cost optical monitoring methods. However, the low accuracy of such micro-stations warrants correction of measured values read from a micro-station. Conventional implementations for such corrections, for example utilization of a fit function of a national optical station and micro-station reading to correct the micro-station, requires a high technical threshold and is not suitable for small instruments such as independent instruments acting as micro-stations due to the dependence on the national network and the user's data processing algorithm. Such an example correct methodology is described in Chinese Patent Number CN110514626A entitled "The Data Calibration Method and Air Pollution Surveillance System of Air Pollution Surveillance System," filed July 23, 2019, the content of which is incorporated herein by reference in its entirety.

Such sample collectors, such as PPB-level gas sensors, are further vulnerable to factors that affect the detection accuracy of such sensors. For example, changes in sensitivity and baseline with changes in ambient temperature and humidity experienced by the sensor and the baseline drift due to use and/or aging of a sample collector. In this regard, the reading error caused by baseline drift can often be significant, even in circumstances where sensitivity compensation and/or baseline calibration is performed. Such reading error reaches unacceptable levels in circumstances where higher resolution sensors, such as PPB-level gas sensors, are utilized in such sample collectors.

Embodiments of the present invention provide for dynamic iterative baseline adjustment. In this regard, the dynamic iterative baseline adjustment updates the value of baseline value(s) utilized by an apparatus, such as a sample collector and/or associated sensor. In some such embodiments, the dynamic iterative baseline adjustment updates each baseline value corresponding to a particular baseline factor interval, (e.g., a temperature interval, humidity interval, or other external condition that affects the baseline value of a sample collector) such that the baseline inaccuracies at different baseline factor intervals are improved. The apparatus and/or associated sensor can utilize the updated baseline values for any of myriad of purposes, such as to correct data values captured and/or otherwise received by the apparatus and/or associated sensor. In this regard, baseline inaccuracies are improved, thus improving the overall accuracy of the end readings output by the apparatus. In this regard, the embodiments described herein advantageously provide improved accuracy at various operational conditions as compared to conventional sensors and/or implementations that attempt baseline corrections. Additionally, the embodiments described herein provide such advantageous without increased technical knowledge and/or implementation requirements by the user.

### Definitions

In some embodiments, some of the operations above may be modified or further amplified. Furthermore, in some embodiments, additional optional operations may be included. Modifications, amplifications, or additions to the operations above may be performed in any order and in any combination.

Many modifications and other embodiments of the invention set forth herein will come to mind to one skilled in the art to which this disclosure pertains having the benefit of the teachings presented in the foregoing description and the associated drawings. Therefore, it is to be understood that the embodiments are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

The term "sample collector" refers to any computing device embodied in hardware, software, firmware, and/or a combination thereof, that collects a sample from a sample environment and processes the sample to perform one or more determinations. In at least one example context, a sample collector is embodied by an electrochemical gas sensor configured for determining a pollutant concentration from a captured gas sample. In at least one other example context, the sample collector is embodied by another system including a chemical sensor for measuring a concentration within a sample. In yet at least one other example context, the sample collector is embodied by another sensor system whose accuracy is affected by user usage over time. In this regard, it should be appreciated that an electrochemical gas sensor is merely one non-limiting example of a sample collector.

The term "measured value" refers to a sensed and/or corrected data value measured by a sensor. In some embodiments, the measured value comprises a PPB-level measurement of a concentration of a particle within a captured sample. For example, in at least one example context, a measured value represents a PPB-level concentration of a particulate, compound, or the like, in a captured air sample. The terms "measured value set" and "set of measured values" refers to one or more data objects including zero or more measured value(s). **In** some embodiments, different measured values are each measured by distinct sensors.

The term "low-point measured value" refers to a measured value representing a numeric value that falls below a corresponding baseline value. The terms "low-point measured value set" and "set of low-point measured value" refers to one or more data objects including zero or more low-point measured value(s).

The term "baseline updating threshold" refers to a threshold number or proportion of measured values that trigger an update of one or more baseline value(s). For example, in some embodiments, the baseline updating threshold represents a percentage threshold wherein, in a circumstance where the proportion of low-point measured value in a measured value set exceeds the baseline updating threshold, a process for updating one or more baseline value(s) is initiated. In some other embodiments, the baseline updating threshold represents a number value where, in a circumstance where the count of low-point measured values in a measured value set exceeds the baseline updating threshold, a process for updating one or more baseline value(s) is initiated.

The term "baseline value" refers to a sensor output when no load is present on the sensor. In some embodiments, the baseline value is represented by a current value driven by the sensor. The terms "baseline value set" and "set of baseline values" refers to one or more data objects including zero or more baseline value(s). In some embodiments, each baseline value of a baseline value set is associated with a different temperature interval of a set of temperature intervals.

The term "corrective baseline algorithm" refers to one or more operations to adjust a baseline value. In some embodiments, a corrective baseline algorithm includes one or more operations to adjust a baseline value to meet an expected value. In some embodiments for example, a corrective baseline algorithm includes one or more operations to ensure the baseline values for a set of temperature intervals meets one or more expected attributes (e.g., is monotonic).

The term "lower sensor limit" refers to a minimum value measurable by a sensor. In some embodiments, for example, a lower sensor limit refers to a minimum PPB value that a particular sensor can measure in a single captured sample.

The term "temperature range" refers to a defined lower temperature limit and upper temperature limit within which a particular sensor is configured for operating, or otherwise determined to be sufficiently accurate and/or otherwise approved for operating.

The term "temperature interval size" refers to a numerical value representing the size of each temperature interval for which a measured value is collected. In some embodiments, a temperature range is segmented into a set of temperature intervals each of the size defined by the temperature interval size. In an example context, for example, a temperature interval size of 5 degrees centigrade is utilized to divide a temperature range into a set of temperature intervals (e.g., for the range of -10C to 10C, the temperature intervals includes (-10C to -5C, -5C to 0C, 0C to 5C, and 5C to 10C). It should be appreciated that embodiments may utilize any temperature interval size, and be configured for any of a myriad of temperature ranges.

The term "factory default baseline" refers to a baseline value associated with a default configuration of a sensor. The terms "factory default baseline set" and "set of factory default baselines" refer to one or more data objects including zero or more factory default baseline(s) provided for any number of temperature intervals. In at least one example context, a factory default baseline represents an initial baseline value at the time a sensor begins operation and has not yet been affected by any baseline changes.

The term "operational conditions" refers to a set of one or more values for environment conditions associated with a particular sample collector that affects one or more baseline value(s) of the sample collector and/or a measured value associated with a collected sample. In some such embodiments, one or more operational conditions are measurable using an associated device or component specifically configured to measure a corresponding operational condition. Non-limiting examples of operational conditions include an operational temperature, humidity, and/or the like, or any combination thereof.

The term "baseline factor interval" refers to a single temperature value or a range of values for an operational condition, or multiple operational conditions, that is utilized to partition a plurality of measured values into one or more groups and/or subsets for processing. In some embodiments, a baseline factor interval is associated with a particular baseline value that is utilized to adjust subsequently measured raw values associated with operational conditions that fall within the associated baseline factor interval. Non-limiting examples of baseline factor intervals include a temperature interval, a humidity interval, and/or the like, or any combination thereof. In an example context where a baseline factor interval comprises a temperature interval, for example, in a circumstance where a measured value is associated with a particular operational temperature, the measured value is grouped to a particular temperature interval based on the operational temperature, and a baseline value corresponding to the temperature interval associated with the measured value.

The term "temperature interval" refers to a single temperature value or range between temperature values. In some embodiments, at least one temperature interval is maintained by an apparatus, such as a sample collector or sensor and/or associated processing circuitry of a sample collector, that is associated with a particular baseline value. In this regard, during operation of the apparatus, a captured sample is determinable as associated with a particular operational temperature that falls within a particular temperature interval maintained by an apparatus. The terms "temperature interval set" and "set of temperature intervals" refer to one or more data objects including zero or more temperature interval(s), which are maintained by one or more apparatuses, devices, sensors and/or associated processing circuitry, or systems.

The term "adjacent baseline factor interval" refers to a particular next determinable baseline factor interval having a set value that is the next higher baseline factor interval or the next lower baseline factor interval in a set of baseline factor intervals. In some embodiments, for example, for a particular baseline factor interval an adjacent baseline factor interval is the baseline factor interval immediately higher than the particular baseline factor interval or the baseline factor interval immediately lower than the particular baseline factor. In some other embodiments where one or more baseline values is interpolated, an adjacent baseline factor interval is a next higher or lower baseline factor interval that has been set to a factor default baseline value or already set via interpolation. In one or more embodiments, the adjacent baseline factor interval is a particular determinable and/or preset temperature interval higher or lower than another temperature interval of a set of temperature intervals. In one example context, a temperature interval of 0C-5C is associated with an adjacent higher temperature interval of 5C-10C, and associated with an adjacent lower temperature interval of -5C-0C.

The term "abnormal measured value" refers to a measured value determined to be erroneous, or otherwise a highest or lowest measured value in a set of measured value. In some embodiments, an abnormal measured value is determinable based on exceeding a maximum threshold value or falling below a minimum expected threshold value. In some such embodiments, an abnormal measured value falls below a minimum sensor limit. It should be appreciated that, in one or more example embodiments, a statistical outlier detection algorithm known in the art is utilized to identify abnormal measured values in a measured value set.

The term "average low-point measured value" refers to a value determined by averaging one or more low-point measured values associated with a particular baseline factor interval. In some embodiments, the average low-point measured value is determined by summing some or all low-point measured values associated with a particular baseline factor interval, and subsequently dividing the sum by the total number of measured values utilized in generating the sum.

The term "raw data value" refers to a data value output by a sensor during operation. The term "current operational baseline factor interval" refers to the temperature interval within which a current temperature associated with the operation of a sensor falls. In one example context, a current temperature of 4C falls within a temperature interval of 0-5C, such that the current operational temperature interval is 0-5C.

The term "corrected data value" refers to a data value adjusted to improve the accuracy of the data value. In some embodiments, a corrected data value refers to a raw data value determined by one or more sensor(s) that is adjusted based on a baseline value corresponding to a current operational baseline factor interval determined and/or otherwise identifiable based on current measured and/or otherwise determined values for operational conditions.

### Example Operational System

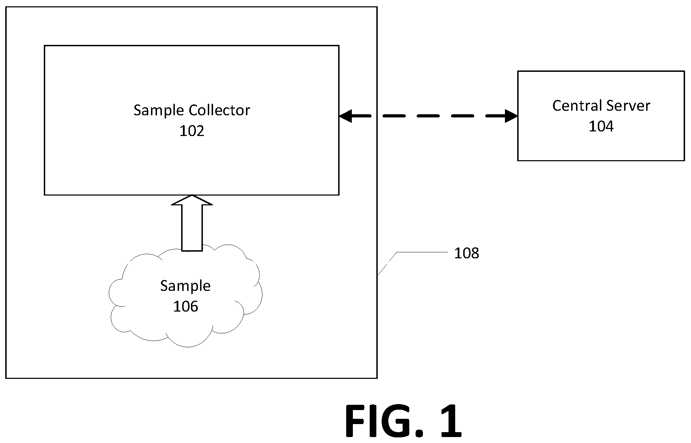

FIG. 1 illustrates an example system in which embodiments of the present invention may operate, in accordance with at least some embodiments of the present invention. As illustrated, the environment includes a sample collector 102. The sample collector 102 is located within a sample environment 108. In this regard, the sample collector 102 may collect samples from the sample environment 108, for example the sample 106. Optionally, in one or more embodiments, the sample collector 102 is communicable over a network with one or more external devices. In other embodiments, the sample collector 102 is communicable over one or more networks with one or more external sample collectors.

In some embodiments, sample collector 102 is embodied by and/or comprises one or more electromechanical and/or electrochemical measuring device(s). In at least one example context, the sample collector 102 comprises a PPB-level gas sensor capable of measuring gas concentrations and/or particulates from a captured sample. For example, the sample collector 102 may be utilized in any of a myriad of atmospheric environment monitoring operations, odor monitoring, toxic gas monitoring, and/or the like. Such operations may be controlled by private and/or public entities in a particular area, environment, and/or the like. For example, in some embodiments, the sample collector 102 is a gas monitoring system that measures pollutant concentration, such as a level of CO, SO2, NO2, O3, VOC, and/or the like, within a captured sample. In some example embodiments, the sample collector 102 comprises a micro-station that utilizes PPB-level gas sensors to determine accurate readings of such pollutants in captured air samples. As illustrated, for example in some embodiments, the sample collector 102 collects and/or otherwise receives the sample 106, which is then analyzed by the sample collector 102 for any of a myriad of determinations, such as the contaminant concentration within the sample 106. It should be appreciated that in some embodiments the sample collector 102 utilizes known sample collector components (e.g., embodied in hardware, software, firmware, and/or a combination thereof) and/or known sample processing components, for example known electrochemical gas sensor implementations, which in some implementations are further specially configured as described herein.

In some embodiments, the sample collector 102 is optionally communicable with a central server 104. In some such embodiments, the sample collector 102 communicates with the central server 104 to retrieve measured values collected and/or aggregated by the central server 104. For example in some contexts, the central server 104 comprises a public database (for example, controlled by a trusted public or private entity) that monitors one or more collector devices throughout one or more environments. In this regard, in some embodiments the sample collector 102 to retrieve measured values representing historical, trusted sample values that have been measured in one or more environments. For example, the sample collector 102 may communicate with the central server 104 to obtain measured values previously captured and/or received by the central server 104 associated with the sample environment 108.

The sample collector 102 may communicate with the central server 104 at one or more specific points in time during configuration, and/or at any time. Additionally or alternatively, in some embodiments the sample collector 102 communicates with the central server 104 to obtain a set of measured values for at least the sample environment 108 either automatically or upon user request. For example, the sample collector 102 may communicate with the central server 104 during an initial configuration of the sample collector 102. In this regard, the sample collector 102 communicates with the central server 104, in some embodiments, when the sample collector 102 is plugged into and/or otherwise communicatively coupled with a user device (such as a personal desktop computer, laptop computer, and/or the like), and/or is plugged into or otherwise communicatively coupled with the central server itself directly. In other embodiments, the sample collector 102 includes networking circuitry embodied in hardware, software, firmware, and/or the like, that enables communication between the sample collector 102 and at least the central server 104. In this regard, in some embodiments, the sample collector 102 is configured to communicate with the central server 104 over a wired or wireless communications network.

### Example Apparatuses of the Invention

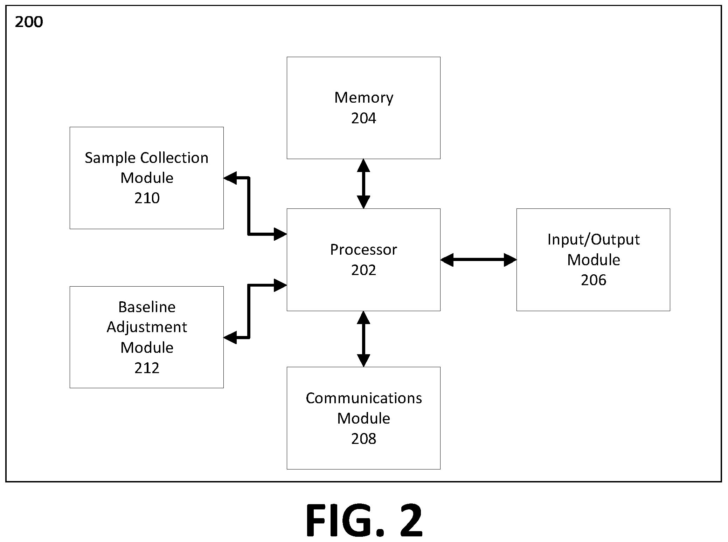

In some embodiments, the sample collector 102 is embodied by one or more computing systems, such as the apparatus 200 shown in FIG. 2. In some embodiments, for example as illustrated, the apparatus 200 includes a processor 202, memory 204, input/output module 206, communications module 208, sample collection module 210, and sample baseline adjustment module 212. In this regard, the apparatus 200 is configured using one or more modules to execute the operations described herein.

Although the components are described with respect to functional limitations, it should be understood that the particular implementations necessarily include the use of particular, specially configured hardware. It should also be understood that certain components of those described herein may include similar or common hardware. For example, in some embodiments, two module both leverage use of the same processor, network interface, storage medium, or the like, to perform their associated functions, such that duplicate hardware is not required for each module. The use of the term "module" and/or the term "circuitry" as used herein with respect to components of the apparatus 200 should therefore be understood to include particular hardware configured to perform the functions associated with the particular module as described herein.

Additionally or alternatively, the terms "module" and "circuitry" should be understood broadly to include hardware and, in some embodiments, software and/or firmware that configures the hardware. For example, in some embodiments, "module" and/or "circuitry" includes processing circuitry, storage media, network interface(s), input/output device(s), and the like. In some embodiments, other elements of the apparatus 200 provide or supplement the functionality of the particular module. For example, in some embodiments, the processor 202 provides processing functionality, the memory 204 provides storage functionality, the communications module 208 provides network interface functionality, and the like, to one or more of the other modules of the apparatus 200.

In some embodiments, the processor 202 (and/or co-processor or any other processing circuitry assisting or otherwise associated with the processor) may be in communication with the memory 204 via a bus for passing information among components of the apparatus 200. In some embodiments, the memory 204 is non-transitory and includes, for example, one or more volatile and/or non-volatile memories. In other words, for example, the memory 204 is embodied by an electronic storage device (e.g., a computer readable storage medium). Additionally or alternatively, in some embodiments, the memory 204 is configured to store information, data, content, applications, instructions, or the like, for enabling the apparatus 200 to carry out various functions in accordance with example embodiments of the present invention. In some embodiments, the memory 204 stores at least a set of measured values (e.g., a historical record of measured values obtained directly or indirectly from a central server) and one or more operational condition values and/or associated baseline factor intervals corresponding to each measured value in the set of measured values, such that the stored data is retrievable for use in one or more of the processing operations described herein.

The processor 202 may be embodied in any one of a myriad of ways and in some embodiments, for example, includes one or more processing devices configured to perform independently. Additionally or alternatively, in some embodiments the processor 202 includes one or more processors configured in tandem via a bus to enable independent execution of instructions, pipelining, and/or multithreading. The use of the terms "processor," "processing module," and "processing circuitry" should be understood to include a single-core processor, a multi-core processor, multiple processors internal to the apparatus 200, and/or remote or "cloud" processors.

In at least one example embodiment, the processor 202 is configured to execute computer-coded instructions stored in the memory 204 and/or another memory otherwise accessible to the processor 202. Alternatively or additionally, in some embodiments the processor 202 is configured to execute hard-coded functionality. As such, whether configured by hardware or software means, or by a combination thereof, the processor 202 represents an entity (e.g., physically embodied in circuitry) capable of performing operations according to an embodiment of the present invention while configured accordingly. Alternatively or additionally, in another example in circumstances where the processor 202 is embodied as an executor of software instructions, the instructions specifically configure the processor 202 to perform the algorithm(s) and/or operation(s) described herein when the instructions are executed via the apparatus 200.

In at least one example context, the processor 202 is configured to enable capturing of a sample from the apparatus 200, for example via one or more sample intake components of the apparatus 200. Such sample intake components may include any of a myriad of known intake components in the art. Additionally or alternatively, in some embodiments the processor 202 is configured to process a captured sample, such as to determine a concentration of pollutants, particulates, and/or the like within the captured sample. In some embodiments, the processor 202 is configured to utilize one or more sub-devices for capturing a sample from the environment, and/or processing the sample, such as by activating a gas sensor, image sensor, and/or the like, and/or receiving and processing the output from such a sensor.

Additionally or alternatively, in some embodiments, the processor 202 is configured to perform a dynamic iterative baseline adjustment algorithm, for example to update one or more baseline values for use in processing sensor output. In some such embodiments, the processor 202 is configured to retrieve one or more measured variables, determining whether the measured variables comprises a set of low-point measured values having a number and/or proportion of measured values exceeds a particular baseline updating threshold, determining updated baseline value(s), and updating the baseline value set to the updated baseline value(s) for each baseline factor interval of a set of baseline factor intervals. Additionally or alternatively, in some embodiments the processor 202 is configured to perform a corrective baseline algorithm to adjust the updated baseline value set. Additionally or alternatively still, in some embodiments, the processor 202 is configured to utilize the updated baseline values to correct one or more measured data values and generate a corrected data value representing a corrected sensor value from a sensor output.

In some embodiments, the apparatus 200 includes input/output module 206 that, alone or in conjunction with processor 202, provides output to the user and/or, in some embodiments, receives an indication of a user input. In some embodiments, the input/output module 206 comprises a preconfigured and/or dynamic user interface, and/or includes a display to which the user interface is rendered. In some embodiments, the input/output module 206 comprises a specially configured input/output application, a web user interface, a mobile application, a desktop application, a linked or networked client device communicable with the apparatus 200 for input and/or output to a user device, or the like. In some embodiments, the input/output module 206 additionally or alternatively includes a keyboard, a mouse, a joystick, a touch screen, touch areas, soft keys, a microphone, a speaker, or other input/output mechanisms. The input/output module 206, alone and/or in conjunction with the processor 202, in some embodiments is configured to control one or more functions of one or more user interface elements through computer program instructions (e.g., software and/or firmware) stored on a memory accessible to the processor (e.g., memory 204, and/or the like). In other embodiments, the input/output module 206 is configured to cause rendering of a predetermined, hard-coded, and/or application-specific user interface specially configured to be output by the apparatus 200 to a display upon execution.

The communications module 208 may be embodied in any means such as a device or circuitry embodied in hardware, software, firmware, and/or any combination thereof, that is configured to receive data from and/or transmit data to a network and/or any other device, circuitry, or module in communication with the apparatus 200. In this regard, in some embodiments, the communications module 208 includes, for example, at least a network interface for enabling communications with a wired or wireless communication network. For example, in some embodiments, the communications module 208 includes one or more network interface cards, antennas, buses, switches, routers, modems, and supporting hardware and/or software, or any other device suitable for enabling communications via a network. Additionally or alternatively, in some embodiments, the communications module 208 includes the circuitry for interacting with antenna(s) and/or other signal transmitter(s), receiver(s), and/or transceiver(s) to cause transmission of signals via such components or to handle receipt of signals received via such components.

In some embodiments, the sample collection module 210 includes hardware, software, firmware, and/or a combination thereof, to support functionality associated with sample collection and processing. In some such embodiments, the sample collection module 210 includes one or more hardware components to enable intake of a sample into the apparatus 200. For example, in some embodiments, the sample collection module 210 includes an intake nozzle, one or more fan(s), air motor(s), and/or other component(s), and/or the like, that enable the sample to be captured from an environment associated with such components. Additionally or alternatively, in some embodiments, the sample collection module 210 includes one or more components that capture and/or enable processing of the sample. For example, in some embodiments, the sample collection module 210 includes a gas sensor, image sensor, and/or other processing circuitry configured to capture data associated with a sample captured in a medium within the sample collection module 210 (e.g., a film, adhesive, and/or the like that captures pollutants within a captured air sample). In some such embodiments, the sample collection module 210, includes hardware, software, and/or firmware sufficient to enable capturing of a sample from an environment and subsequent processing of said sample. It should be appreciated that, in some embodiments, the sample collection module 210 may include a separate processor, specially configured field programmable gate array (FPGA), or a specially configured application-specific integrated circuit (ASIC).

The baseline adjustment module 212 includes hardware, software, firmware, and/or a combination thereof, to support functionality associated with dynamic iterative baseline adjustments. The baseline adjustment module 212 includes hardware, software, firmware, and/or a combination thereof, that determines, from a set of measured values, a number of low-point measured values that exceeds a baseline updating threshold, such as where each measured value in the set of measured values is associated with a particular baseline factor interval of a set of baseline factor intervals, and where each baseline factor interval of the set of baseline factor intervals is associated with a baseline value of a baseline value set, and where the set of measured values comprises a subset of low-point measured values comprising each measured value that is lower than the baseline value for the baseline factor interval corresponding to the measured value. Additionally, the baseline adjustment module 212 includes hardware, software, firmware, and/or a combination thereof, that determines an updated baseline value set comprising an updated baseline value for each baseline factor interval of a set of baseline factor intervals, and updates the baseline value set to an updated baseline value set by, for each baseline factor interval of the set of baseline factor intervals, updating the baseline value of the baseline value set associated with the temperature interval to the updated baseline value associated with the baseline factor interval. Additionally the baseline adjustment module 212 includes hardware, software, firmware, and/or a combination thereof, that performs a corrective baseline algorithm on the updated baseline value set, and optionally measures a raw data value associated with a current operational baseline factor interval, and optionally generates a corrected data value based on (1) the raw data value and (2) an updated baseline value associated with the current operational baseline factor interval. Additionally or alternatively still, optionally in some embodiments, the baseline adjustment module 212 includes hardware, software, firmware, and/or a combination thereof, that determines, based on the set of measured values, a number and/or proportion of measured values that fall below a lower sensor limit, and/or sets the baseline updating threshold based on the number and/or proportion of measured values that fall below the lower sensor limit. Additionally or alternatively still, optionally in some embodiments, the baseline adjustment module 212 includes hardware, software, firmware, and/or a combination thereof, that segments a range of baseline factor values into a set of baseline factor intervals based on a predetermined and/or determined baseline factor interval size. For example, in one or more embodiments, the baseline adjustment module 212 includes hardware, software, firmware, and/or a combination thereof, that segments a temperature range into the set of temperature intervals based on a temperature interval size, and for at least one temperature interval of the set of temperature intervals. Additionally or alternatively, in some embodiments, the baseline adjustment module 212 includes hardware, software, firmware, and/or a combination thereof, that assigns a factory default baseline as the baseline value for one or more baseline factor interval(s) of the set of baseline factor intervals, assigns the baseline value based on a linear interpolation between a first baseline value for a first baseline factor interval and a second baseline value for a second baseline factor interval, where the first baseline factor interval is an adjacent lower baseline factor interval and the second baseline factor interval is an adjacent higher baseline factor interval. In some embodiments, the baseline adjustment module 212 performs one or more of such operations in conjunction with one or more components of the apparatus 200, for example the processor 202, memory 204, input/output module 206, communications module 208, and/or sample collection module 210. It should be appreciated that, in some embodiments, the baseline adjustment module 212 may include a separate processor, specially configured FPGA, or a specially configured ASIC.

In one or more example embodiments, the apparatus 200 is embodied by an existing sensor system specially configured for performing dynamic iterative baseline adjustments as described herein. For example, in some such embodiments, the apparatus 200 is specially configured such that processing circuitry (e.g., processor 202) associated with a sample processing sensor (e.g., an electrochemical sensor), and/or the sample processor sensor itself, is configured to perform the dynamic iterative baseline adjustments described herein automatically at predetermined intervals and/or upon specific input. In this regard, some such embodiments are embodied by existing hardware specially configured by specialized software instructions for performing the operations described herein.

In some embodiments, one or more of the aforementioned components is combined to form a single module. For example, in some embodiments, the sample collection module 210 and baseline adjustment module 212 are combined into a single module. Additionally or alternatively, the baseline adjustment module 212 and/or sample collection module 210 are combined with the processor 202. The combined module may be configured to perform some or all of the functionality described above with respect to the individual modules. Additionally or alternatively, in some embodiments, one or more of the modules described above may be configured to perform one or more of the actions described with respect to one or more of the other modules.

### Example Processes of the Invention

Having described example apparatuses and interfaces for initiating specific processes, example flowcharts including various operations performed by apparatuses, devices, and/or subsystems of the above described systems will now be discussed. It should be appreciated that each of the flowcharts depicts an example computer-implemented process that may be performed by one, or more, of the above described apparatuses, systems, or devices. In regard to the below flowcharts, one or more of the depicted operational blocks may be optional in some, or all, embodiments. Optional operational blocks are depicted with broken (dashed) lines.

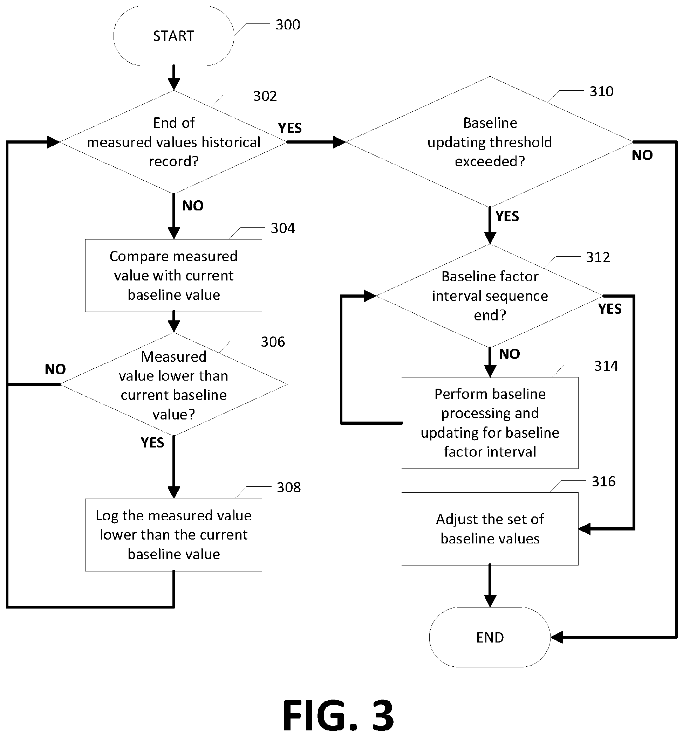

It should be appreciated that the particular operations depicted and described herein with respect to FIGS. 3-9 illustrate specific operations or steps of a particular process. Further in this regard, the process is implementable by computer hardware, software, firmware, and/or a combination thereof, of a system, apparatus, device, and/or the like, as a computer-implemented method. In other embodiments, the various blocks represent operations capable of being performed by an apparatus, device, system, and/or the like. For example, computer-coded instructions may be specially programmed for performing the various operations depicted and stored for execution by an apparatus, for example in one or more memory device(s) of the apparatus for execution by one or more processor(s) of the apparatus. In other embodiments, computer program product(s) are provided that are capable of executing the operations depicted by various blocks. For example, in some embodiments, a computer program product includes one or more non-transitory memory devices and/or other computer-readable storage media having computer program code stored thereon that, in execution with a processor, apparatus, and/or the like, is configured for performing the operations depicted in the process(es).

FIG. 3 illustrates an example process for dynamic iterative baseline adjustment, in accordance with at least one embodiment of the present invention. The example process 300 illustrated may be performed by any of the devices described herein. For example, in one or more embodiments, the process depicted is performed by a sample collector 102 embodied by the apparatus 200.

As illustrated, the process 300 begins at operation 302. At operation 302, the apparatus 200 includes means, such as the baseline adjustment module 212, sample collection module 210, communications module 208, input/output module 206, processor 202, and/or the like, to determine whether the end of a historical record of measured values has been reached. In some such embodiments, the apparatus 200 is configured to maintain, identify, and/or otherwise retrieve the historical record of measured values (e.g., a set of measured values). The historical set of measured values may represent values for previously measured samples, such as by a central trusted system and/or devices associated therewith. In this regard, in at least one context the historical record of measured values represents trusted measured values for use in adjusting the baseline values maintained by the apparatus 200. In this regard, in some embodiments, the apparatus 200 retrieves the historical record set of measured values, and iterates through each historical measured value by proceeding to operation 304 until all historical records of a measured value have been processed.

In a circumstance where end of the historical record is not reached, the historical record comprises a subsequent measured value to be processed and flow continues to operation 304. At operation 304, the apparatus 200 includes means, such as the baseline adjustment module 212, sample collection module 210, communications module 208, input/output module 206, processor 202, and/or the like, to compare the measured value with a current baseline value. In some such embodiments, each record of a measured value comprises and/or otherwise is associated with one or more particular baseline factor interval(s) associated with operational conditions within which the sample was measured. In some such embodiments, the apparatus 200 identifies a baseline factor interval associated with the record of the measured value, and utilizes the baseline factor interval to identify the appropriate corresponding baseline value for comparison. For example, in some embodiments, the apparatus 200 maintains a set of current baseline values associated with a set of baseline factor intervals, such that the apparatus 200 may identify a current baseline value from the set of current baseline values that corresponds to the appropriate baseline factor interval for a particular historical record. In some such embodiments, the apparatus 200 compares the measured value with the current baseline value to determine whether the measured value represents a value below the current baseline value, or whether the measured value represents a value equal to or greater than the current baseline value.

At operation 306, the apparatus 200 includes means, such as the baseline adjustment module 212, sample collection module 210, communications module 208, input/output module 206, processor 202, and/or the like, to determine whether the measured value is lower than the current baseline value. As illustrated, in a circumstance where the measured value is determined not lower than the associated current baseline value, flow returns to operation 302. In this regard, the apparatus 200 begins to process the next measured value of the historical record if one exists. Otherwise, in a circumstance where the measured value determines the measured value is lower than the associated current baseline value, flow proceeds to operation 308.

At operation 308, the apparatus 200 includes means, such as the baseline adjustment module 212, sample collection module 210, communications module 208, input/output module 206, processor 202, and/or the like, to log the measured value is lower than the current baseline value. In some embodiments, the apparatus 200 maintains a counter or other data value that represents the number of measured values lower than their corresponding current baseline value. In some such embodiments, the apparatus 200 increments the counter at operation 308. In other embodiments, the apparatus 200 marks the record representing the measured data value as lower than the current baseline value, such that the apparatus 200 may determine the total number of measured values determined low than their associated current baseline value based on the number of marked records upon processing all measured values in the historical log. Upon completion of operation 308, flow returns to operation 302 for processing the next record of a measured value if one exists.

At operation 302, in a circumstance where the apparatus 200 determines the end of the measured values historical record is reached (e.g., all measured values in the set of measured values have been processed according to the operations 302-308), flow proceeds to operation 310. At operation 310, the apparatus 200 includes means, such as the baseline adjustment module 212, sample collection module 210, communications module 208, input/output module 206, processor 202, and/or the like, to determine whether a baseline updating threshold is exceeded. For example, in some embodiments, the apparatus 200 determines whether a proportion of low-point measured values in the measured value set (e.g., measured values lower than their corresponding baseline value) exceeds the baseline updating threshold, such as where the baseline updating threshold represents a proportional threshold. In other embodiments, the apparatus 200 determines whether the count of low-point measured values (e.g., the measured values that are lower than the associated current baseline value) exceeds a baseline updating threshold, such as where the baseline updating threshold represents a numerical count threshold. In this regard, the baseline updating threshold may represent a number or proportion of low-point measured values that are determined from a set of measured values before adjustment of one or more baseline values is initiated. For example, in some embodiments, the count of measured values lower than the associated baseline value for that measured value is maintained, and the count or proportion of low-point measured values is determined to exceed the baseline updating threshold, the apparatus initiates one or more operations of a dynamic iterative baseline value updating process to update each baseline value in a baseline value set from a current value to an updated baseline value, as described herein. The baseline updating threshold is set in any of the myriad of manners described herein.

In a circumstance where the baseline updating threshold is not exceeded by the count or proportion of measured values lower than the associated current baseline value for the baseline factor interval does not exceed a baseline updating threshold, the flow ends. In some such circumstances, the apparatus 200 determines that a baseline update is not needed, such that the apparatus 200 continues to utilize the current baseline values. In some embodiments, upon ending the flow, the apparatus 200 waits for a predetermined length of time, waits until occurrence of a predetermined evet, and/or otherwise initiates a subsequent process for dynamic iterative baseline updating, for example via the processes described herein. For example, in some embodiments, the apparatus 200 initiates the process monthly, bi-weekly, weekly, or at another pre-determined time interval. It should be appreciated that the apparatus 200 may be utilized to capture and/or process one or more samples while waiting for the next process for dynamic iterative baseline adjustment to begin.

In a circumstance where the baseline updating threshold is not exceeded by the count or proportion of measured values lower than the associated current baseline value for the measured value, flow proceeds to operation 312. At operation 312, the apparatus 200 includes means, such as the baseline adjustment module 212, sample collection module 210, communications module 208, input/output module 206, processor 202, and/or the like, to determine whether the end of a baseline factor interval sequence has been reached. In this regard, the set of measured values retrieved, identified, and/or otherwise maintained by the apparatus 200 is associated with a set of baseline factor intervals, such that the apparatus 200 further maintains and/or otherwise stores a baseline value associated with each baseline factor interval of the set of baseline factor intervals. In some embodiments, the apparatus 200 iterates through each baseline factor interval of the set of baseline factor intervals to update the current baseline value associated with each baseline factor interval. In this regard, in a circumstance where the apparatus 200 determines the baseline factor interval sequence end has not been reached, the flow continues to operation 314 for processing the next baseline factor interval in the baseline factor interval sequence.

At operation 314, the apparatus 200 includes means, such as the baseline adjustment module 212, sample collection module 210, communications module 208, input/output module 206, processor 202, and/or the like, to perform baseline processing and updating for the next baseline factor interval. In this regard, in some embodiments the apparatus 200 identifies measured values of a set of measured values that are associated with the next baseline factor interval, such that the identified measured values associated with the particular baseline factor interval may be processed for purpose of updating the baseline value associated with the baseline factor interval. In some embodiments, the apparatus 200 processes the low-point measured values associated with the particular baseline factor interval to determine a new, updated baseline value. In various embodiments, the apparatus 200 utilizes one or more algorithms for determining an updated baseline value based on some or all of the low-point measured values associated with the particular baseline factor interval. In at least one example embodiment, the apparatus 200 determines an average measured value from one or more low-point measured values associated with the baseline factor interval for setting as the updated baseline value associated with the baseline factor interval. It should be appreciated that, in some such embodiments, the apparatus 200 repeats such processing for each baseline factor interval in the set of baseline factor intervals.

At operation 316, the apparatus 200 includes means, such as the baseline adjustment module 212, sample collection module 210, communications module 208, input/output module 206, processor 202, and/or the like, to adjust the baseline value set. In some such embodiments, the apparatus 200 performs a corrective baseline algorithm on the updated baseline value set. In this regard, in some such embodiments, the updated baseline value set is corrected to meet an expected value, property (e.g., defined by an expected physical property or relation between the values of the updated baseline value set), and/or the like. In some such embodiments, the set of adjusted updated baseline value is utilized as the new, updated baseline value set for use. In some embodiments, the apparatus 200 stores the adjusted updated baseline value set for further utilization, as described herein.

In some embodiments, the process 300 is again initiated upon completion of operation 316. In some such embodiments, the apparatus 200 continues to perform the process 300 until operation 310 is determined not satisfied (e.g., the baseline updating threshold is not exceeded). In some such embodiments, the baseline value set is iteratively updated until the current baseline values represented by the baseline value set are sufficient for utilization by the apparatus 200. In some such embodiments, the iterative manner of performing dynamic baseline adjustments prevents over-adjusting the baseline values and similarly prevents under-adjusting the baseline values. In this regard, the described processes for dynamic iterative baseline adjustment represents an improved methodology over conventional processes for adjusting baseline value(s).

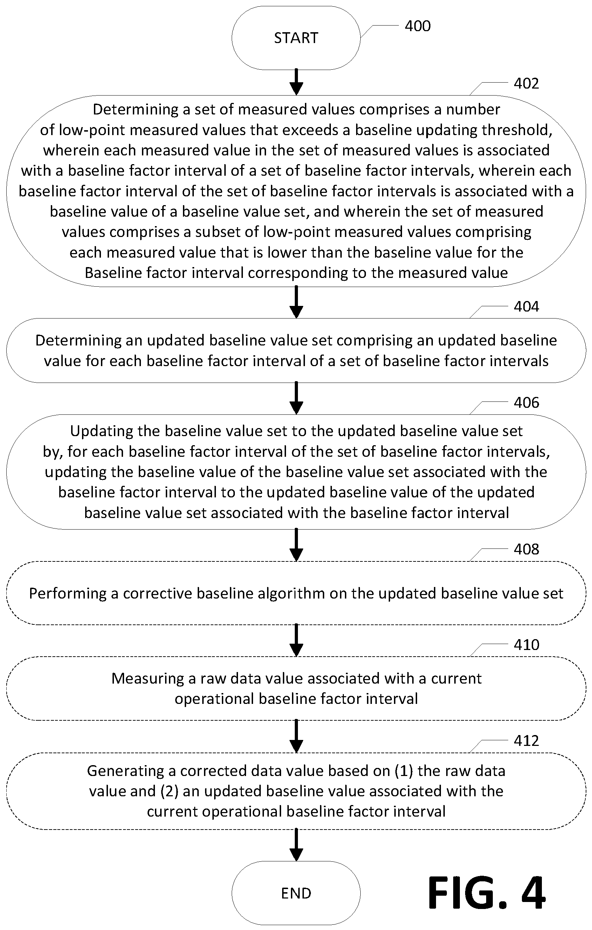

FIG. 4 illustrates an example process for dynamic iterative baseline adjustment, in accordance with at least one embodiment of the present invention. The example process 400 illustrated may be performed by any of the devices described herein. For example, in one or more embodiments, the process 300 is performed by a sample collector 102 embodied by the apparatus 200.

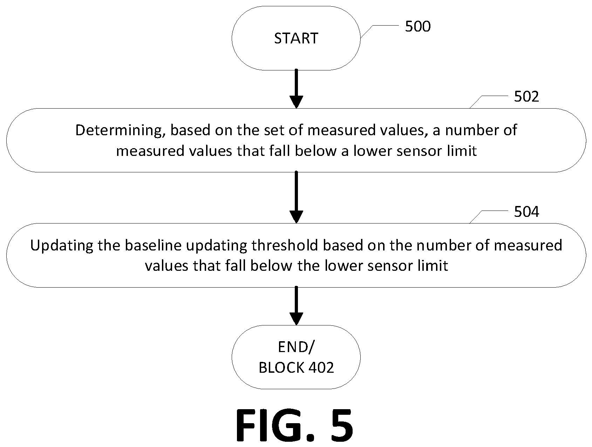

Process 400 begins at operation 402. At operation 402, the apparatus 200 includes means, such as the baseline adjustment module 212, sample collection module 210, communications module 208, input/output module 206, processor 202, and/or the like, to determine a set of measured values comprises a number of low-point measured values that exceeds a baseline updating threshold. In some such embodiments, the baseline updating threshold is set in any of a myriad of ways. For example, in some embodiments, the baseline updating threshold is set via a process described herein, for example as described with respect to FIG. 5. In other embodiments, the baseline updating threshold is set based on a user input value. In yet other embodiments, the baseline updating threshold is derived from one or more measured values utilizing any of a myriad of algorithms for processing said one or more measured values.

In some embodiments, the apparatus 200 identifies and/or otherwise retrieves the set of measured values stored by the apparatus 200, where each measured value includes and/or is otherwise associated with a particular baseline factor interval, such that each baseline factor interval in a set of baseline factor intervals defines a subset of measured values. In some such embodiments, the apparatus 200 determines the subset of low-point measured values for each baseline factor interval of the set of baseline factor intervals and/or a number of low-point measured values for each baseline factor interval of the set of baseline factor intervals. For example, in some embodiments, the apparatus 200 compares each measured value associated with a particular baseline factor interval with a baseline value associated with the particular baseline factor interval, wherein the measured value is identified as a low-point measured value in a circumstance where the value of the measured value is lower than the corresponding baseline value. In this regard, in some such embodiments, the apparatus 200 repeats such processing for each baseline factor interval of the set of baseline factor intervals to identify the subset of low-point measured values for all baseline factor intervals of the set of baseline factor intervals. The apparatus 200 may identify that the number and/or proportion of low-point measured values exceeds the baseline updating threshold based on the subset of low-point measured values.

At operation 404, the apparatus 200 includes means, such as the baseline adjustment module 212, sample collection module 210, communications module 208, input/output module 206, processor 202, and/or the like, to determine an updated baseline value set comprising an updated baseline value for each baseline factor interval of a set of baseline factor intervals. **In** some such embodiments, the updated baseline value set includes an updated baseline value for each baseline factor interval corresponding to a measured value in the set of measured values. **In** one or more example embodiments, the updated baseline value for any baseline factor interval is determined based on a subset of the set of measured values, such as one or more measured values associated with that baseline factor interval. In some such embodiments, the apparatus 200 identifies a subset of the measured values including each low-point measured value associated with the particular baseline factor interval, and processes the low-point measured values associated with the particular baseline factor interval to determine the corresponding updated baseline value for that particular baseline factor interval. For example, in at least one example embodiment, the apparatus 200 performs at least one algorithm based on the identified subset of measured values associated with the particular baseline factor interval to determine the updated baseline value for that particular baseline factor interval, as described herein. In some such embodiments, the apparatus 200 determines the updated baseline value for each baseline factor interval of the set of baseline factor intervals in this manner.

At operation 406, the apparatus 200 includes means, such as the baseline adjustment module 212, sample collection module 210, communications module 208, input/output module 206, processor 202, and/or the like, to update the baseline value set to the updated baseline value set by, for each baseline factor interval of the set of baseline factor intervals, updating the baseline value of the baseline value set associated with the baseline factor interval to the updated baseline value of the updated baseline value set associated with the baseline factor interval. In this regard, the apparatus 200 updates a current baseline value stored and/or otherwise maintained by the apparatus 200 associated with a particular baseline factor interval to the value represented by the updated baseline value associated with that particular baseline factor interval, and does so for each baseline factor interval of the set of baseline factor intervals. In some embodiments, for example, the apparatus 200 maintains a baseline factor-baseline table that maps each baseline factor interval of the set of baseline factor intervals to a particular current value representing the baseline value. In some such embodiments, the apparatus 200 updates the current baseline value for each baseline factor value to the corresponding updated baseline value of the updated baseline set. In this regard, the stored baseline factor-baseline table represents the updated values of the updated baseline value set for subsequent retrieval and/or use by the apparatus 200. In other embodiments, one or more data objects is maintained by the apparatus 200 representing the updated baseline value set for subsequent retrieval and/or use by the apparatus 200.

At operation 408, the apparatus 200 includes means, such as the baseline adjustment module 212, sample collection module 210, communications module 208, input/output module 206, processor 202, and/or the like, to perform a corrective baseline algorithm on the updated baseline value set. In some such embodiments, the corrective baseline algorithm adjusts the value of one or more of the updated baseline values. In some such embodiments, one or more of the updated baseline values is/are updated such that the updated baseline value and/or set of updated baseline values meets one or more expected properties, relationships, and/or the like. For example, in some embodiments, the corrective baseline algorithm ensures the updated baseline value set maintains an expected monotonic relationship. It should be appreciated that in some embodiments the apparatus 200 is configured to perform one or more corrective baseline algorithm(s) for any number of expected relationships, properties, and/or the like. In some embodiments, the apparatus 200 performs the corrective baseline algorithm on the updated baseline value set utilizing the stored baseline factor-baseline table stored and/or otherwise maintained by the apparatus 200. In at least one example context, the corrective baseline algorithm is specifically configured to ensure the updated baseline value set meets at least one natural law, for example a fundamental law of electrochemistry.

It should be appreciated that the updated baseline value set may be utilized for any of a myriad of operations. At optional operation 410, the apparatus 200 includes means, such as the baseline adjustment module 212, sample collection module 210, communications module 208, input/output module 206, processor 202, and/or the like, to measure a raw data value associated with a current operational baseline factor interval. In some embodiments, the apparatus 200 comprises one or more gas sensors, intake components, and/or the like, to enable intake of a sample and/or processing of a sample to determine a raw data value. In some such embodiments, the raw data value represents one or more properties processed from the sample. For example, in some contexts, the raw data value represents a concentration of a particular particulate within a captured and/or otherwise received sample. Additionally or alternatively, in some embodiments, the apparatus 200 includes a baseline factor measurer that determines the operational baseline factor interval with which the raw data value is associated. For example, in some embodiments, the apparatus 200 determines that a captured sample associated with the raw data value was captured at particular operating conditions within a corresponding operational baseline factor interval. In this regard, in some such embodiments, the apparatus 200 determines the particular operational baseline factor is within the current operational baseline factor interval maintained by the apparatus 200. In other embodiments, the apparatus 200 receives a data value representing the operational baseline factor at which the sample was captured and determines the current operational baseline factor interval from the received operational baseline factor associated with the captured sample. Alternatively or additionally, in some embodiments, the apparatus 200 receives the operational baseline factor interval associated with a captured sample associated with the raw data value.

At optional operation 412, the apparatus 200 includes means, such as the baseline adjustment module 212, sample collection module 210, communications module 208, input/output module 206, processor 202, and/or the like, to generate a corrected data value based on (1) the raw data value and (2) an updated baseline value associated with the current operational baseline factor interval. In some such embodiments, the raw data value is adjusted based on the updated baseline value associated with the current operational baseline factor interval such that the generated corrected data value accurately represents the measured value at the current operational baseline factor interval. In this regard, the corrected data value represents the raw data value adjusted to account for inaccuracies in the raw data value, for example due to baseline drift and/or other inaccuracies affecting the sensor utilized to measure the raw data value. In this regard, the corrected data value represents the true value of the property to be measured with improved accuracy over the raw data value.

In some embodiments, the apparatus 200 is configured to output the corrected data value to a display, such that the user of the apparatus 200 may view the corrected data value for example. In other embodiments, the apparatus 200 further processes the corrected data value before outputting any data to a display. For example, in one example context where the apparatus 200 embodies a gas sensor, the apparatus 200 divides the corrected data value by a sensor sensitivity at the corresponding baseline factor interval, and outputs the result to the display. In this regard for example, in some embodiments, the apparatus 200 outputs the corrected data value or any of a number of associated data values derived therefrom.

FIG. 5 illustrates example additional operations of an example process for dynamic iterative baseline adjustment, in accordance with at least one embodiment of the present invention. The example process 500 illustrated may be performed by any of the devices described herein. For example, in one or more embodiments, the process depicted is performed by a sample collector 102 embodied by the apparatus 200.

Process 500 begins at operation 502. In some embodiments, flow returns to one or more other operations upon completion of the process 500. For example, as illustrated, in some embodiments, flow proceeds to operation 402 as depicted and described with respect to the process 400 herein upon completion of the operation 504. In other embodiments, flow may end or proceed to another operation of one or more of the flows described herein.

At operation 502, the apparatus 200 includes means, such as the baseline adjustment module 212, sample collection module 210, communications module 208, input/output module 206, processor 202, and/or the like, to determine, based on the set of measured values, a number of measured values that fall below a lower sensor limit. In some such embodiments, the apparatus 200 compares each measured value with the lower sensor limit to determine whether the measured value falls below the lower sensor limit. Additionally or alternatively, in some such embodiments, the apparatus 200 marks and/or otherwise flags measured values that fall below the lower sensor limit such that the number of measured values that fall below the lower sensor limit is determinable based on the marked measured values. Additionally or alternatively, in some embodiments, the apparatus 200 stores and/or otherwise maintains a data value representing a count of the number of measured values that fall below the lower sensor limit, and increments the count when a measured value in the set of measured value is compared with the lower sensor limit to determine the measured value is lower than the lower sensor limit. In this regard, the count represents the number of measured values that fall below the lower sensor limit upon completion of the iteration for all measured values in the set of measured values. Additionally or alternatively, the count representing the number of measured values that fall below the lower sensor limit is usable to derive a proportion of such measured values compared to the total number of measured values in a particular set of measured values.

In some embodiments, the lower sensor limit represents a value below which a particular sensor in and/or otherwise associated with the apparatus 200 cannot function or is otherwise determined not to function with sufficient accuracy. For example, in one example context, the apparatus 200 comprises and/or otherwise is associated with a gas sensor that determines particular concentration, and the lower sensor limit represents a minimum particulate concentration that the gas sensor is configured to identify. In some such embodiments, the apparatus 200 determines the lower sensor limit from the associated sensor. In other embodiments, the apparatus 200 receives (for example, as user input or through communication with another device) the lower sensor limit of an associated sensor, and stores and/or otherwise maintains the lower sensor limit for subsequent retrieval.

At operation 504, the apparatus 200 includes means, such as the baseline adjustment module 212, sample collection module 210, communications module 208, input/output module 206, processor 202, and/or the like, to update the baseline updating threshold based on the number of measured values that fall below the lower sensor limit. In some such embodiments, the apparatus 200 sets a value for the baseline updating threshold that equals the number of measured values that fall below the lower sensor limit. Alternatively or additionally, in some embodiments, the apparatus 200 sets a value for the baseline updating threshold that equals a proportion of the number of measured values that fall below the lower sensor limit to the total number of measured values (e.g., a proportion of measured values below the sensor limit). In this regard, in some such embodiments, the apparatus 200 utilizes the updated baseline updating threshold in one or more subsequent determinations, for example to determine whether or not to initiate one or more processes for dynamic iterative adjustment as described herein, such as at operation 310 of the process 300 described herein.

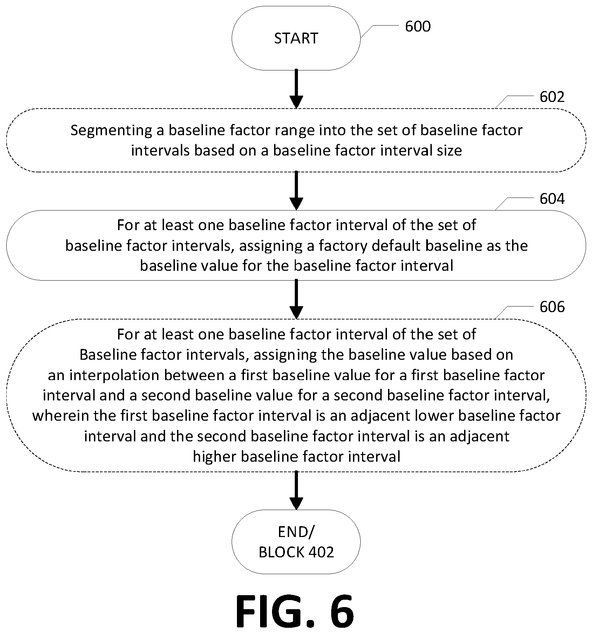

FIG. 6 illustrates example additional operations of an example process for dynamic iterative baseline adjustment, specifically for setting default baseline values in accordance with the present invention. The example process 600 illustrated may be performed by any of the devices described herein. For example, in one or more embodiments, the process depicted is performed by a sample collector 102 embodied by the apparatus 200.

Process 600 begins at optional operation 602. In some embodiments, flow returns to one or more other operations upon completion of the process 600. For example, as illustrated, in some embodiments, flow proceeds to operation 402 as depicted and described with respect to the process 400 herein upon completion of the operation 606. In other embodiments, flow may end or proceed to another operation of one or more of the flows described herein.

At optional operation 602, the apparatus 200 includes means, such as the baseline adjustment module 212, sample collection module 210, communications module 208, input/output module 206, processor 202, and/or the like, to segment a baseline factor range into a set of baseline factor intervals based on a baseline factor interval size. In some embodiments, the baseline factor interval size is predetermined and/or otherwise maintained by the apparatus 200. In other embodiments, the apparatus 200 receives the baseline factor interval size, for example automatically from another device during configuration of the apparatus 200, and/or the apparatus receives the baseline factor interval size in response to user input data. In some such embodiments, a user of the apparatus 200 inputs the baseline factor interval size to the apparatus 200 for use in segmenting a baseline factor range. In yet other embodiments, the apparatus 200 is preconfigured comprising the set of baseline factor intervals without such segmenting.

At operation 604, the apparatus 200 includes means, such as the baseline adjustment module 212, sample collection module 210, communications module 208, input/output module 206, processor 202, and/or the like, to, for at least one baseline factor interval of the set of baseline factor intervals, assign a factory default baseline as the baseline value for the baseline factor interval. In some such embodiments, the apparatus 200 receives at least one factory default baseline for at least one baseline factor interval from another device, such as during configuration of the apparatus 200. In other embodiments, the apparatus 200 receives the at least one factory default baseline for at least one baseline factor interval in response to user input from a user of the apparatus 200 and/or another device communicatively coupled with the apparatus 200. Additionally or alternatively, in some embodiments, the apparatus 200 is preconfigured to store and/or otherwise maintain the one or more factory default baseline(s) for the at least one baseline factor interval of the set of baseline factor intervals.

In some embodiments, the baseline value associated with each baseline factor interval of the set of baseline factor intervals is assigned to a factory default baseline. In other embodiments, one or more of the baseline factor intervals is not set to a factory default baseline. At operation 606, the apparatus 200 includes means, such as the baseline adjustment module 212, sample collection module 210, communications module 208, input/output module 206, processor 202, and/or the like, to, for at least one baseline factor interval of the set of baseline factor intervals, assign the baseline value based on an interpolation between (1) a first baseline value for a first baseline factor interval and (2) a second baseline value for a second baseline factor interval, for example where the first baseline factor interval is an adjacent lower baseline factor interval and where the second baseline factor interval is an adjacent higher baseline factor interval. The one or more baseline values is determined based on a linear interpolation between the adjacent lower baseline factor interval and the adjacent higher baseline factor interval. In some embodiments, a baseline value determined through interpolation is utilized in a subsequent interpolation to assign another baseline value. In other embodiments, other interpolation algorithms are utilized to assign one or more baseline value(s) of the baseline value set. In one or more of such embodiments, the apparatus 200 utilizes the assigned baseline values in one or more operations described herein.

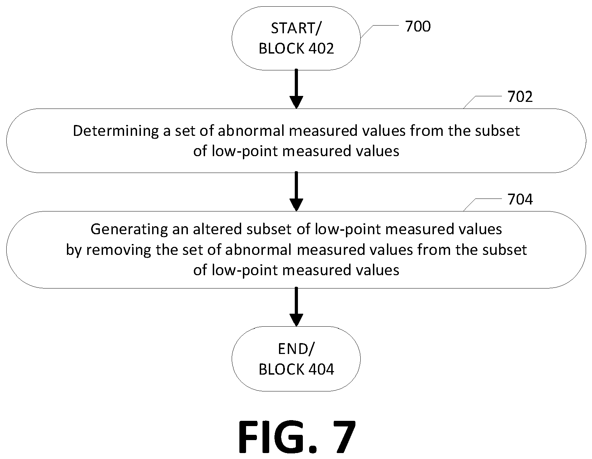

FIG. 7 illustrates example additional operations of an example process for dynamic iterative baseline adjustment, specifically for altering a subset of low-point measured values by removing a set of abnormal measured values in accordance with at least one embodiment of the present invention. The example process 700 illustrated may be performed by any of the devices described herein. For example, in one or more embodiments, the process depicted is performed by a sample collector 102 embodied by the apparatus 200.

Process 700 begins at operation 702. In some embodiments, flow returns to one or more other operations upon completion of the process 600. For example, as illustrated, in some embodiments, flow proceeds to operation 702 as depicted and described with respect to the process 400 herein upon completion of the operation 606. In other embodiments, flow may end or proceed to another operation of one or more of the flows described herein.

At operation 702, the apparatus 200 includes means, such as the baseline adjustment module 212, sample collection module 210, communications module 208, input/output module 206, processor 202, and/or the like, to determine a set of abnormal measured values from the subset of low-point measured values. In some embodiments, the apparatus 200 determines the set of abnormal measured values by determining one or more measured values that represent measurements outside the operational range of a sensor within and/or associated with the apparatus 200. Additionally or alternatively, in some embodiments, the apparatus 200 determines the set of abnormal measured values by identifying all measured values that fall below a lower sensor limit associated with a sensor within and/or associated with the apparatus 200. Additionally or alternatively still in some embodiments, the apparatus 200 determines the set of abnormal measured values utilizing one or more abnormal value detection algorithms. For example, in some embodiments, the apparatus 200 determines the set of abnormal values by identifying measured values that are above a threshold value from an expected value, limit value, and/or the like.

In at least one embodiment, the apparatus 200 utilizes a predetermined proportion of maximum measured value(s) and minimum measured value(s) to determine one or more measured values of the set of abnormal measured values. For example, in some embodiments, a predetermined proportion of maximal measured values (e.g., the highest measured values in a measured value set) are determined as abnormal measured values, and/or the same proportion or a second predetermined proportion of minimum measured values (e.g. the lowest measured values in the measured value set) are similarly determined as abnormal measured values. Additionally or alternatively, in some embodiments, a determinable and/or predetermined standard deviation of measured values in a measured value set is utilized for determining one or more abnormal measured values in the measured value set. For example, in one or more embodiments, measured values that fall outside of a predetermined number of standard deviations from a mean measured value for the measured value set and/or median measured value for the measured value set are determined as anomaly measured value(s) to be removed from consideration. In one example context, measured values that are more than one (1) standard deviation from the mean measured value for a measured value set and/or one (1) standard deviation from the median measured value for the measured value set are determined as abnormal measured values. In this regard, in one or more example contexts, the abnormal measured values are removed from consideration for purposes of generating an updated baseline value set.

At operation 704, the apparatus 200 includes means, such as the baseline adjustment module 212, sample collection module 210, communications module 208, input/output module 206, processor 202, and/or the like, to generate an altered subset of low-point measured values. In some such embodiments, the altered subset of low-point measured values is generated by removing the set of abnormal measured values from the subset of low-point measured values. In this regard, the altered subset of low-point measured values removes consideration of the abnormal measured values, which in some contexts includes extreme values not to be considered for one or more subsequent determinations performed by the apparatus 200. For example, in some such embodiments, the altered subset of low-point measured values is utilized for determining and/or otherwise updating one or more baseline values based on one or more low-point measured values for a baseline factor interval, for example at operation 404 of the process 400 described herein. It should be appreciated that, in some embodiments, "removing" each abnormal measured value does not physically require moving and/or deleting data from one or more data object(s), and in some embodiments is accomplished by marking each abnormal measured value with a data flag indicating the measured value as an abnormal measured value to remove it from further consideration as part of the subset of low-point measured values.

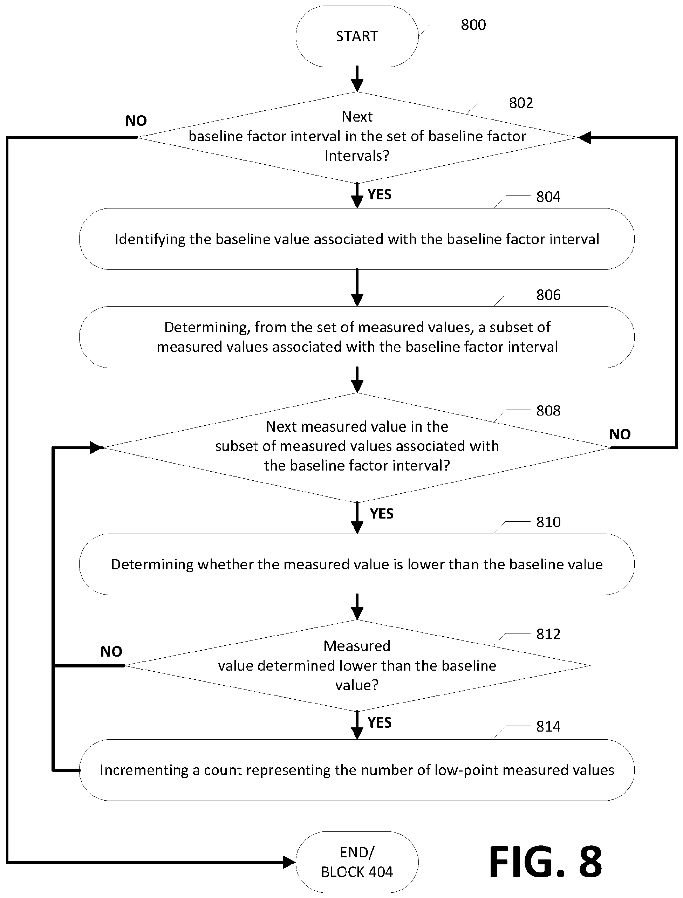

FIG. 8 illustrates example additional operations of an example process for dynamic iterative baseline adjustment, specifically for determining a set of measured values comprises a number of low-point measured values that exceeds a baseline updating threshold in accordance with at least one embodiment of the present invention. The example process 800 illustrated may be performed by any of the devices described herein. For example, in one or more embodiments, the process depicted is performed by a sample collector 102 embodied by the apparatus 200.

Process 800 begins at operation 802. In some embodiments, flow returns to one or more other operations upon completion of the process 600. For example, as illustrated, in some embodiments, flow proceeds to operation 702 as depicted and described with respect to the process 400 herein upon completion of the operation 606. In other embodiments, flow may end or proceed to another operation of one or more of the flows described herein.

At operation 802, the apparatus 200 includes means, such as the baseline adjustment module 212, sample collection module 210, communications module 208, input/output module 206, processor 202, and/or the like, to determine whether a next baseline factor interval exists in the set of baseline factor intervals. In this regard, in some such embodiments, the apparatus 200 iterates through each baseline factor interval in the set of baseline factor intervals. In some such embodiments, the apparatus 200 performs one or more operations for the next particular baseline factor interval to be processed, for example one or more of the operations 804-814. In this regard, flow proceeds to operation 804 in a circumstance where the apparatus 200 determines a next baseline factor interval exists in the set of baseline factor intervals. The flow ends or proceeds to an operation of another process, such as operation 404 of the process 400 described herein, in a circumstance where the apparatus 200 does not identify a next baseline factor interval in the set of baseline factor intervals.

At operation 804, the apparatus 200 includes means, such as the baseline adjustment module 212, sample collection module 210, communications module 208, input/output module 206, processor 202, and/or the like, to identify the baseline value associated with the baseline factor interval. In some such embodiments, the apparatus 200 determines the current value of a baseline value associated with the particular baseline factor interval being processed. For example, in some embodiments, the apparatus 200 maintains and/or otherwise stores a baseline factor-baseline table, such that the apparatus 200 identifies the baseline value associated with the baseline factor interval by retrieving the current value in the baseline factor-baseline table for that particular baseline factor interval.

At operation 806, the apparatus 200 includes means, such as the baseline adjustment module 212, sample collection module 210, communications module 208, input/output module 206, processor 202, and/or the like, to determine, from the set of measured values, a subset of measured values associated with the baseline factor interval. In some embodiments, each measured value of the measured value set is stored associated with a particular baseline factor interval associated with a sample corresponding to the measured value. In some such embodiments, the apparatus 200 queries and/or otherwise searches the set of measured values for measured values that are associated with the particular baseline factor interval being processed. In this regard, the apparatus 200 may determine any number of measured values associated with the particular baseline factor interval being processed.

At operation 808, the apparatus 200 includes means, such as the baseline adjustment module 212, sample collection module 210, communications module 208, input/output module 206, processor 202, and/or the like, to determine whether a next measured value exists in the subset of measured values associated with the baseline factor interval being processed. In this regard, in some such embodiments, the apparatus 200 iterates through each measured value associated with the baseline factor interval being processed, for example one or more of the operations 810-814. In some such embodiments, the apparatus 200 performs one or more operations for the next particular baseline factor interval to be processed, for example one or more of the operations 804-814. In this regard, flow proceeds to operation 810 in a circumstance where the apparatus 200 determines a next measured value exists in the subset of measured values associated with the baseline factor interval being processed, for example to process the next identified measured value. The flow returns to operation 802 in a circumstance where the apparatus 200 does not identify a next measured value in the subset of measured values.

At operation 810, the apparatus 200 includes means, such as the baseline adjustment module 212, sample collection module 210, communications module 208, input/output module 206, processor 202, and/or the like, to determine whether the measured value is lower than the baseline value associated with the baseline factor interval being processed. In some such embodiments, the apparatus 200 compares the measured value with the baseline value to determine whether the measured value is lower than the baseline value. It should be appreciated that each measured value may result differently based on the comparison between each measured value and the baseline value.

At operation 808, the apparatus 200 includes means, such as the baseline adjustment module 212, sample collection module 210, communications module 208, input/output module 206, processor 202, and/or the like, to determine whether the measured value was determined lower than the baseline value for the baseline factor interval being processed. In some such embodiments, such a determination is made based on the results of the operation 810. In this regard, in some such embodiments, flow proceeds to operation 814 in a circumstance where the apparatus 200 determines the measured value is lower than the baseline value for the baseline factor interval being processed. In this regard, the apparatus 200 continues processing subsequent measured values if such measured values exist. The flow returns to operation 808 in a circumstance where the apparatus 200 determines the measured value is not lower than the baseline value being processed.

At operation 814, the apparatus 200 includes means, such as the baseline adjustment module 212, sample collection module 210, communications module 208, input/output module 206, processor 202, and/or the like, to increment a count representing the number of low-point measured values. In some such embodiments, the apparatus 200 maintains the count representing the number of low-point measured values as a data object having a particular value. In this regard, the count may be incremented by adding to the current value of the count. In other embodiments, the apparatus 200 increments the count representing the number of low-point measured values by marking the measured value, and/or an associated record, using one or more data flags. In some such embodiments, the apparatus 200 determines the count of the number of low-point measured values by identifying the number of measured values in the measured value set marked as lower than their corresponding baseline value. Upon incrementing the count, flow returns to operation 808, for example for subsequent processing of a next measured value for the baseline factor interval if one exists, or to proceed to processing for a subsequent baseline factor interval if one exists.

In this regard, in some embodiments, the operations of process 800 are repeated such that the measured values associated with each baseline factor interval is processed and considered. In some such embodiments, the apparatus 200 processes the set of measured values such that the count represents the total number of low-point measured values for the entire set of measured values. In some embodiments, the apparatus 200 utilizes the count representing the number of low-point measured values in the set of measured values for one or more operations, for example the determination at operation 404 of the process 400 as described herein.

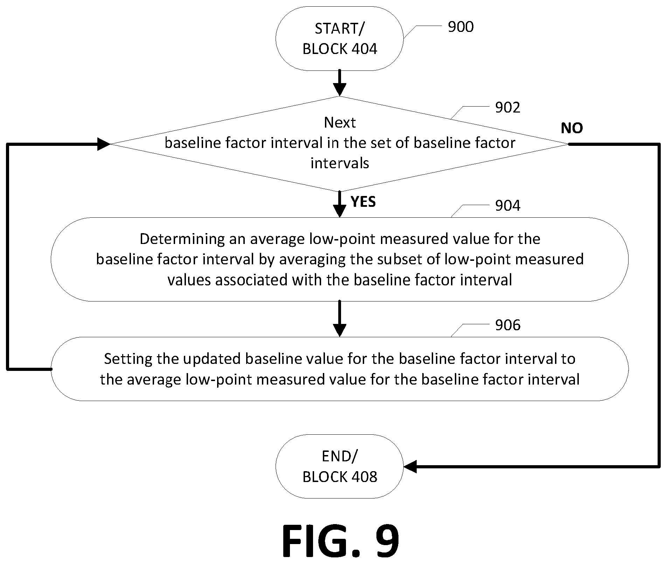

FIG. 9 illustrates example additional operations of an example process for dynamic iterative baseline adjustment, specifically for determining an updated baseline value set in accordance with at least one embodiment of the present invention. The example process 900 illustrated may be performed by any of the devices described herein. For example, in one or more embodiments, the process depicted is performed by a sample collector 102 embodied by the apparatus 200.

Process 900 begins at operation 902. In some embodiments, flow returns to one or more other operations upon completion of the process 900. For example, as illustrated, in some embodiments, flow proceeds to optional operation 408 as depicted and described with respect to the process 400 herein upon completion of the operation 902. In other embodiments, flow may end or proceed to another operation of one or more of the flows described herein.

At operation 902, the apparatus 200 includes means, such as the baseline adjustment module 212, sample collection module 210, communications module 208, input/output module 206, processor 202, and/or the like, to determine whether a next baseline factor interval exists in the set of baseline factor intervals. In this regard, in some such embodiments, the apparatus 200 iterates through each baseline factor interval in the set of baseline factor intervals. In some such embodiments, the apparatus 200 performs one or more operations for the next particular baseline factor interval to be processed, for example one or more of the operations 904-906. In this regard, flow proceeds to operation 904 in a circumstance where the apparatus 200 determines a next baseline factor interval exists in the set of baseline factor intervals. The flow ends or proceeds to an operation of another process, such as optional operation 408 of the process 400 described herein, in a circumstance where the apparatus 200 does not identify a next baseline factor interval in the set of baseline factor intervals.

At operation 904, the apparatus 200 includes means, such as the baseline adjustment module 212, sample collection module 210, communications module 208, input/output module 206, processor 202, and/or the like, to determine an average low-point measured value for the baseline factor interval. In some such embodiments, the apparatus 200 determines the average low-point measured value for the baseline factor interval by averaging the subset of low-point measured values associated with the baseline factor interval. In this regard, in some such embodiments, the apparatus 200 identifies one or more low-point measured values associated with the baseline factor interval, for example the measured values that are lower than the baseline value corresponding to the baseline factor interval. In some such embodiments, the apparatus 200 averages all low-point measured values associated with the particular baseline factor interval. In other embodiments, the apparatus 200 averages a subset of the low-point measured values associated with the baseline factor interval being processed. For example, in some embodiments, the apparatus 200 removes one or more abnormal measured values before determining the average low-point measured value, as described herein. In this regard, in some such embodiments, the apparatus 200 determines the average low-point measured value for the baseline factor interval based on an altered subset of low-point measured values associated with the baseline factor interval being processed.

At operation 906, the apparatus 200 includes means, such as the baseline adjustment module 212, sample collection module 210, communications module 208, input/output module 206, processor 202, and/or the like, to set the updated baseline value for the baseline factor interval to the average low-point measured value for the baseline factor interval. In some such embodiments, the apparatus 200 stores the average low-point measured value for the baseline factor interval as the value for the baseline value associated with the baseline factor interval being processed. In some such embodiments, the apparatus 200 updates a baseline-baseline factor table to include the average low-point measured value associated with the baseline factor interval being processed. For example, in some such embodiments, the apparatus 200 updates a data record of the baseline factor-baseline table that is associated with the baseline factor interval being processed such that the data records includes and/or otherwise represents the average low-point measured value as the updated baseline value.

In some embodiments, flow returns to operation 902. In this regard, in some such embodiments, the apparatus 200 repeats for all baseline factor intervals of the set of baseline factor intervals. In this regard, each baseline value associated with a baseline factor interval of the set of baseline factor intervals is set to an updated baseline value. In one or more embodiments, the updated baseline value(s) representing an updated baseline value set are utilized in one or more operations, for example optional operation 408 of the process 400 as described herein.

### Conclusion

Although an example processing system has been described above, implementations of the subject matter and the functional operations described herein can be implemented in other types of digital electronic circuitry, or in computer software, firmware, or hardware, including the structures disclosed in this specification and their structural equivalents, or in combinations of one or more of them.

Embodiments of the subject matter and the operations described herein can be implemented in digital electronic circuitry, or in computer software, firmware, or hardware, including the structures disclosed in this specification and their structural equivalents, or in combinations of one or more of them. Embodiments of the subject matter described herein can be implemented as one or more computer programs, i.e., one or more modules of computer program instructions, encoded on computer storage medium for execution by, or to control the operation of, information/data processing apparatus. Alternatively, or in addition, the program instructions can be encoded on an artificially-generated propagated signal, e.g., a machine-generated electrical, optical, or electromagnetic signal, which is generated to encode information/data for transmission to suitable receiver apparatus for execution by an information/data processing apparatus, such as a sample collector. A computer storage medium can be, or be included in, a computer-readable storage device, a computer-readable storage substrate, a random or serial access memory array or device, or a combination of one or more of them. Moreover, while a computer storage medium is not a propagated signal, a computer storage medium can be a source or destination of computer program instructions encoded in an artificially-generated propagated signal. The computer storage medium can also be, or be included in, one or more separate physical components or media (e.g., multiple CDs, disks, or other storage devices).

The operations described herein can be implemented as operations performed by an information/data processing apparatus on information/data stored on one or more computer-readable storage devices or received from other sources. Alternatively or additionally, the operations can be implemented as operations of a computer-implemented method. Alternatively or additionally, the operations can be implemented as operations performed by one or more apparatus(es) and/or system(s) embodied in hardware, software, firmware, and/or a combination thereof.

The term "data processing apparatus" encompasses all kinds of apparatus, devices, and machines for processing data, including by way of example a programmable processor, a computer, a system on a chip, or multiple ones, or combinations, of the foregoing. The apparatus can include special purpose logic circuitry, e.g., an FPGA or an ASIC. The apparatus can also include, in addition to hardware, code that creates an execution environment for the computer program in question, e.g., code that constitutes processor firmware, a protocol stack, a repository management system, an operating system, a cross-platform runtime environment, a virtual machine, or a combination of one or more of them. The apparatus and execution environment can realize various different computing model infrastructures, such as web services, distributed computing and grid computing infrastructures. The apparatus can additionally or alternatively include specialized hardware for sample collection and/or processing, for example such that the apparatus embodies a sample collector for a particular type of sample medium (e.g., a gas sensor, liquid sensor, and/or the like). The sample collection and/or processing hardware, software, and/or firmware can be embodied in any of a myriad of manners known in the art for such purposes, and in some embodiments, be configured to function utilizing the dynamic iterative baseline adjustment processes described herein.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or information/data (e.g., one or more scripts stored in a markup language document), in a single file dedicated to the program in question, or in multiple coordinated files (e.g., files that store one or more modules, sub-programs, or portions of code). A computer program can be deployed to be executed on one computer or on multiple computers or computing device(s) that are located at one site or distributed across multiple sites and interconnected by a communication network.

The processes and logic flows described herein can be performed by one or more programmable processors executing one or more computer programs to perform actions by operating on input information/data and generating output. Processors suitable for the execution of a computer program include, by way of example, both general and special purpose microprocessors, and any one or more processors of any kind of digital computer. Generally, a processor will receive instructions and information/data from a read-only memory or a random access memory or both. The essential elements of a computer are a processor for performing actions in accordance with instructions and one or more memory devices for storing instructions and data. Generally, a computer will also include, or be operatively coupled to receive information/data from or transfer information/data to, or both, one or more mass storage devices for storing data, e.g., magnetic, magneto-optical disks, or optical disks. However, a computer need not have such devices. Devices suitable for storing computer program instructions and information/data include all forms of non-volatile memory, media and memory devices, including by way of example semiconductor memory devices, e.g., EPROM, EEPROM, and flash memory devices; magnetic disks, e.g., internal hard disks or removable disks; magneto-optical disks; and CD-ROM and DVD-ROM disks. The processor and the memory can be supplemented by, or incorporated in, special purpose logic circuitry. Additionally or alternatively still, as described herein, the computer can include specialized sample collection and/or processing hardware, software, and/or firmware.

To provide for interaction with a user, embodiments of the subject matter described herein can be implemented on a computer having a display device, e.g., a CRT (cathode ray tube) or LCD (liquid crystal display) monitor, for displaying information/data to the user and user input buttons, displays, and/or peripherals, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's client device in response to requests received from the web browser.

Certain features that are described herein in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the embodiments described above should not be understood as requiring such separation in all embodiments, and it should be understood that the described program components and systems can generally be integrated together in a single software product or packaged into multiple software products.

Thus, particular embodiments of the subject matter have been described. Other embodiments are within the scope of the following claims. In some cases, the actions recited in the claims can be performed in a different order and still achieve desirable results. In addition, the processes depicted in the accompanying figures do not necessarily require the particular order shown, or sequential order, to achieve desirable results. In certain implementations, multitasking and parallel processing may be advantageous.

## Claims

1. A computer-implemented method of calibration of baseline values of a gas sensor,
comprising:
determining (402), from a set of measured values, a number of low-point measured values that exceeds a baseline updating threshold, wherein each measured value in the set of measured values is associated with a baseline factor interval of a set of baseline factor intervals, wherein each baseline factor interval of the set of baseline factor intervals is associated with a baseline value of a baseline value set, and wherein the set of measured values comprises a subset of low-point measured values comprising each measured value that is lower than the baseline value for the baseline factor interval corresponding to the measured value;
determining (404) an updated baseline value set comprising an updated baseline value for each baseline factor interval of the set of baseline factor intervals, wherein the updated baseline value for each baseline factor interval of the set of baseline factor intervals is determined based on at least one low-point measured value of the subset of low-point measured values that is associated with the baseline factor interval;
updating (406) the baseline value set to the updated baseline value set by, for each baseline factor interval of the set of baseline factor intervals, updating the baseline value of the baseline value set associated with the baseline factor interval to the updated baseline value associated with the baseline factor interval; and
performing (408) a corrective baseline algorithm on the updated baseline value set,
wherein the method further comprises: for at least one baseline factor interval of the set of baseline factor intervals, assigning (604) a factory default baseline as the baseline value for the baseline factor interval; and for at least one baseline factor interval of the set of baseline factor intervals, assigning (606) the baseline value based on a linear interpolation between a first baseline value for a first baseline factor interval and a second baseline value for a second baseline factor interval, wherein the first baseline factor interval is an adjacent lower baseline factor interval and the second baseline factor interval is an adjacent higher baseline factor interval.

2. The method according to claim 1, wherein performing (408) the corrective
baseline algorithm comprises:
determining a monotonic trend of the updated baseline value set based on a change between each updated baseline value of the updated baseline value set for a change between each baseline factor interval in the set of baseline factor intervals.

3. The method according to any one of claims 1-2, the method further comprising:
determining (502), based on the set of measured values, a number of measured values that fall below a lower sensor limit; and
setting (504) the baseline updating threshold based on the number of measured values that fall below the lower sensor limit.

4. The method (400) according to any one of claims 1-3, the method further comprising:
setting the baseline value set by:
segmenting (602) a baseline factor range into the set of baseline factor intervals based on an baseline factor interval size.

5. The method according to any one of claims 1-4, wherein determining (402) the number of low-point measured values that exceeds the baseline updating threshold comprises:
for each baseline factor interval of the set of baseline factor intervals:
identifying (804) the baseline value associated with the baseline factor interval;
determining (806), from the set of measured values, a subset of measured values associated with the baseline factor interval; and
for each measured value in the subset of measured values associated with the baseline factor interval:
determining (810) whether the measured value is lower than the baseline value; and
in a circumstance where the measured value is lower than the baseline value, incrementing (814) a count representing the number of low-point measured values.

6. The method according to any one of claims 1-5, the method further comprising:
determining (702) a set of abnormal measured values from the subset of low-point measured values; and
generating (704) an altered subset of low-point measured values by removing the set of abnormal measured values from the subset of low-point measured values,
wherein the updated baseline value set is determined based on the altered subset of low-point measured values.

7. The method according to any one of claims 1-6, wherein determining (404) the updated baseline value set comprises:
for each baseline factor interval in the set of baseline factor intervals:
determining (904) an average low-point measured value for the baseline factor interval by averaging the subset of low-point measured values associated with the baseline factor interval; and
setting (906) the updated baseline value for the baseline factor interval to the average-low point measured value for the baseline factor interval.

8. The method according to any one of claims 1-7, the method further comprising:
measuring (410) a raw data value associated with a current operational baseline factor interval; and
generating (412) a corrected data value based on:
the raw data value, and
an updated baseline value associated with the current operational baseline factor interval.

9. The method according to any one of claims 1-8, wherein the set of measured values comprises historical measured values received from a central server or plurality of connected devices.

10. The method according to any one of claims 1-9, wherein the method is initiated upon waiting a pre-determined time interval.

11. The method according to any one of claims 1-10, wherein the method is initiated upon occurrence of a predetermined event.

12. The method according to any one of claims 1-11, wherein the method further comprises storing a baseline factor-baseline table comprising each baseline value for each baseline factor interval.

13. An apparatus (200) comprising at least one processor (202) and at least one memory (204) including computer program code, the at least one memory (204) and the computer program code configured to, with the at least one processor (202), cause the apparatus (200) to perform any one of the methods of claims 1-12.

14. A computer program product comprising at least one non-transitory computer-readable medium having computer-readable program instructions stored therein, the computer-readable program instructions comprising instructions, which when performed by an apparatus (200), are configured to cause the apparatus (200) to at least perform any one of the methods of claims 1-12.

## Patentansprüche

1. Computerimplementiertes Verfahren einer Kalibrierung von Basislinienwerten eines Gassensors, umfassend:
Bestimmen (402), aus einem Satz von Messwerten, einer Anzahl von Niedrigpunkt-Messwerten, die einen Basislinienaktualisierungsschwellenwert überschreiten, wobei jeder Messwert in dem Satz von Messwerten einem Basislinienfaktorintervall eines Satzes von Basislinienfaktorintervallen zugehörig ist, wobei jedes Basislinienfaktorintervall des Satzes von Basislinienfaktorintervallen einem Basislinienwert eines Basislinienwertsatzes zugehörig ist, und wobei der Satz von Messwerten einen Teilsatz von Niedrigpunkt-Messwerten umfasst, der jeden Messwert umfasst, der niedriger als der Basislinienwert für das dem Messwert entsprechende Basislinienfaktorintervall ist;
Bestimmen (404) eines aktualisierten Basislinienwertsatzes, der einen aktualisierten Basislinienwert für jedes Basislinienfaktorintervall des Satzes von Basislinienfaktorintervallen umfasst, wobei der aktualisierte Basislinienwert für jedes Basislinienfaktorintervall des Satzes von Basislinienfaktorintervallen auf Basis von mindestens einem Niedrigpunkt-Messwert des Teilsatzes von Niedrigpunkt-Messwerten bestimmt wird, der dem Basislinienfaktorintervall zugehörig ist;
Aktualisieren (406) des Basislinienwertsatzes auf den aktualisierten Basislinienwertsatz durch Aktualisieren, für jedes Basislinienfaktorintervall des Satzes von Basislinienfaktorintervallen, des Basislinienwertes des dem Basislinienfaktorintervall zugehörigen Basislinienwertsatzes auf den dem Basislinienfaktorintervall zugehörigen aktualisierten Basislinienwert; und
Durchführen (408) eines korrigierenden Basislinienalgorithmus an dem aktualisierten Basislinienwertsatz,
wobei das Verfahren ferner Folgendes umfasst: für mindestens ein Basislinienfaktorintervall des Satzes von Basislinienfaktorintervallen, Zuordnen (604) einer Werkseinstellungsbasislinie als den Basislinienwert für das Basislinienfaktorintervall; und für mindestens ein Basislinienfaktorintervall des Satzes von Basislinienfaktorintervallen, Zuordnen (606) des Basislinienwertes auf Basis einer linearen Interpolation zwischen einem ersten Basislinienwert für ein erstes Basislinienfaktorintervall und einem zweiten Basislinienwert für ein zweites Basislinienfaktorintervall, wobei das erste Basislinienfaktorintervall ein benachbartes niedrigeres Basislinienfaktorintervall ist und das zweite Basislinienfaktorintervall ein benachbartes höheres Basislinienfaktorintervall ist.

2. Verfahren nach Anspruch 1, wobei das Durchführen (408) des korrigierenden Basislinienalgorithmus Folgendes umfasst:
Bestimmen einer monotonen Entwicklung des aktualisierten Basislinienwertsatzes auf Basis einer Veränderung zwischen jedem aktualisierten Basislinienwert des aktualisierten Basislinienwertsatzes für eine Veränderung zwischen jedem Basislinienfaktorintervalls in dem Satz von Basislinienfaktorintervallen.

3. Verfahren nach einem der Ansprüche 1 bis 2, das Verfahren ferner umfassend:
Bestimmen (502), auf Basis des Satzes von Messwerten, einer Anzahl von Messwerten, die unter einer niedrigeren Sensorgrenze liegen; und
Festlegen (504) des Basislinienaktualisierungsschwellenwertes auf Basis der Anzahl von Messwerten, die unter der niedrigeren Sensorgrenze liegen.

4. Verfahren (400) nach einem der Ansprüche 1 bis 3, das Verfahren ferner umfassend:
Festlegen des Basislinienwertsatzes durch:
Segmentieren (602) eines Basislinienfaktorbereichs in den Satz von Basislinienfaktorintervallen auf Basis einer Basislinienfaktorintervallgröße.

5. Verfahren nach einem der Ansprüche 1 bis 4, wobei das Bestimmen (402) der Anzahl von Niedrigpunkt-Messwerten, die den Basislinienaktualisierungsschwellenwert überschreiten, Folgendes umfasst:
für jedes Basislinienfaktorintervall des Satzes von Basislinienfaktorintervallen:
Identifizieren (804) des Basislinienwertes, der dem Basislinienfaktorintervall zugehörig ist;
Bestimmen (806), aus dem Satz von Messwerten, eines dem Basislinienfaktorintervall zugehörigen Teilsatzes von Messwerten; und
für jeden Messwert in dem dem Basislinienfaktorintervall zugehörigen Teilsatz von Messwerten:
Bestimmen (810), ob der Messwert niedriger als der Basislinienwert ist; und
in einem Fall, wo der Messwert niedriger als der Basislinienwert ist, Erhöhen (814) einer Zahl, die die Anzahl von Niedrigpunkt-Messwerten darstellt.

6. Verfahren nach einem der Ansprüche 1 bis 5, das Verfahren ferner umfassend:
Bestimmen (702) eines Satzes von anomalen Messwerten aus dem Teilsatz von Niedrigpunkt-Messwerten; und
Erzeugen (704) eines geänderten Satzes von Niedrigpunkt-Messwerten durch Entfernen des Satzes von anomalen Messwerten aus dem Teilsatz von Niedrigpunkt-Messwerten,
wobei der aktualisierte Basislinienwertsatz auf Basis des geänderten Teilsatzes von Niedrigpunkt-Messwerten bestimmt wird.

7. Verfahren nach einem der Ansprüche 1 bis 6, wobei das Bestimmen (404) des aktualisierten Basislinienwertsatzes Folgendes umfasst:
für jedes Basislinienfaktorintervall in dem Satz von Basislinienfaktorintervallen:
Bestimmen (904) eines mittleren Niedrigpunkt-Messwertes für das Basislinienfaktorintervall durch Mitteln des dem Basislinienfaktorintervall zugehörigen Teilsatzes von Niedrigpunkt-Messwerten; und
Festlegen (906) des aktualisierten Basislinienwertes für das Basislinienfaktorintervall auf den mittleren Niedrigpunkt-Messwert für das Basislinienfaktorintervall.

8. Verfahren nach einem der Ansprüche 1 bis 7, das Verfahren ferner umfassend:
Messen (410) eines Rohdatenwertes, der einem aktuellen betrieblichen Basislinienfaktorintervall zugehörig ist; und
Erzeugen (412) eines korrigierten Datenwertes auf Basis von Folgendem:
dem Rohdatenwert, und
einem aktualisierten Basislinienwert, der dem aktuellen betrieblichen Basislinienfaktorintervall zugehörig ist.

9. Verfahren nach einem der Ansprüche 1 bis 8, wobei der Satz von Messwerten historische Messwerte umfasst, die von einem zentralen Server oder einer Vielzahl von verbundenen Vorrichtungen empfangen werden.

10. Verfahren nach einem der Ansprüche 1 bis 9, wobei das Verfahren nach Abwarten eines vorherbestimmten Zeitintervalls eingeleitet wird.

11. Verfahren nach einem der Ansprüche 1 bis 10, wobei das Verfahren nach Auftreten eines vorherbestimmten Ereignisses eingeleitet wird.

12. Verfahren nach einem der Ansprüche 1 bis 11, wobei das Verfahren ferner das Speichern einer Basislinienfaktor-Basislinientabelle umfasst, die jeden Basislinienwert für jedes Basislinienfaktorintervall umfasst.

13. Einrichtung (200), umfassend mindestens einen Prozessor (202) und mindestens einen Speicher (204), der Computerprogrammcode beinhaltet, wobei der mindestens eine Speicher (204) und der Computerprogrammcode dazu konfiguriert sind, mit dem mindestens einen Prozessor (202) die Einrichtung (200) zu veranlassen, eines der Verfahren der Ansprüche 1 bis 12 durchzuführen.

14. Computerprogrammprodukt, umfassend mindestens ein nicht transitorisches, computerlesbares Medium, das darauf gespeicherte computerlesbare Programmanweisungen aufweist, wobei die computerlesbaren Programmanweisungen Anweisungen umfassen, die bei Durchführung durch eine Einrichtung (200) dazu konfiguriert sind, die Einrichtung (200) zu veranlassen, mindestens eines der Verfahren der Ansprüche 1 bis 12 durchzuführen.

## Revendications

1. Procédé mis en œuvre par ordinateur pour l'étalonnage de valeurs de référence d'un capteur de gaz, comprenant :
la détermination (402), à partir d'un ensemble de valeurs mesurées, d'un nombre de valeurs mesurées basses qui dépasse un seuil de mise à jour de référence, dans lequel chaque valeur mesurée dans l'ensemble de valeurs mesurées est associée à un intervalle de facteurs de référence d'un ensemble d'intervalles de facteurs de référence, dans lequel chaque intervalle de facteurs de référence de l'ensemble d'intervalles de facteurs de référence est associé à une valeur de référence d'un ensemble de valeurs de référence, et dans lequel l'ensemble de valeurs mesurées comprend un sous-ensemble de valeurs mesurées basses comprenant chaque valeur mesurée qui est inférieure à la valeur de référence pour l'intervalle de facteurs de référence correspondant à la valeur mesurée ;
la détermination (404) d'un ensemble de valeurs de référence mises à jour comprenant une valeur de référence mise à jour pour chaque intervalle de facteurs de référence de l'ensemble d'intervalles de facteurs de référence, dans lequel la valeur de référence mise à jour pour chaque intervalle de facteurs de référence de l'ensemble d'intervalles de facteurs de référence est déterminée sur la base d'au moins une valeur mesurée basse du sous-ensemble de valeurs mesurées basses qui est associée à l'intervalle de facteurs de référence ;
la mise à jour (406) de l'ensemble de valeurs de référence vers l'ensemble de valeurs de référence mises à jour en mettant à jour, pour chaque intervalle de facteurs de référence de l'ensemble d'intervalles de facteurs de référence, la valeur de référence de l'ensemble de valeurs de référence associée à l'intervalle de facteurs de référence vers la valeur de référence mise à jour associée à l'intervalle de facteurs de référence ; et
l'exécution (408) d'un algorithme de correction de référence sur l'ensemble de valeurs de référence mises à jour,
dans lequel le procédé comprend en outre : pour au moins un intervalle de facteurs de référence de l'ensemble d'intervalles de facteurs de référence, l'attribution (604) d'une référence par défaut d'usine comme valeur de référence pour l'intervalle de facteurs de référence ; et pour au moins un intervalle de facteurs de référence de l'ensemble d'intervalles de facteurs de référence, l'attribution (606) de la valeur de référence sur la base d'une interpolation linéaire entre une première valeur de référence pour un premier intervalle de facteurs de référence et une deuxième valeur de référence pour un deuxième intervalle de facteurs de référence, dans lequel le premier intervalle de facteurs de référence est un intervalle de facteurs de référence inférieur adjacent et le deuxième intervalle de facteurs de référence est un intervalle de facteurs de référence supérieur adjacent.

2. Procédé selon la revendication 1, dans lequel l'exécution (408) de l'algorithme de correction de référence comprend :
la détermination d'une tendance monotone de l'ensemble de valeurs de référence mises à jour sur la base d'une variation entre chaque valeur de référence mise à jour de l'ensemble de valeurs de référence mises à jour pour une variation entre chaque intervalle de facteurs de référence dans l'ensemble d'intervalles de facteurs de référence.

3. Procédé selon l'une quelconque des revendications 1 à 2, le procédé comprenant en outre :
la détermination (502), sur la base de l'ensemble de valeurs mesurées, d'un nombre de valeurs mesurées qui se situent en dessous d'une limite inférieure du capteur ; et
le réglage (504) du seuil de mise à jour de référence sur la base du nombre de valeurs mesurées qui se situent en dessous de la limite inférieure du capteur.

4. Procédé (400) selon l'une quelconque des revendications 1 à 3, le procédé comprenant en outre :
le réglage de l'ensemble de valeurs de référence par :
la segmentation (602) d'une plage de facteurs de référence en l'ensemble d'intervalles de facteurs de référence sur la base d'une taille d'intervalle de facteurs de référence.

5. Procédé selon l'une quelconque des revendications 1 à 4, dans lequel la détermination (402) du nombre de valeurs mesurées basses qui dépassent le seuil de mise à jour de référence comprend :
pour chaque intervalle de facteurs de référence de l'ensemble d'intervalles de facteurs de référence :
l'identification (804) de la valeur de référence associée à l'intervalle de facteurs de référence ;
la détermination (806), à partir de l'ensemble de valeurs mesurées, d'un sous-ensemble de valeurs mesurées associées à l'intervalle de facteurs de référence ; et
pour chaque valeur mesurée dans le sous-ensemble de valeurs mesurées associées à l'intervalle de facteurs de référence :
la détermination (810) du fait que la valeur mesurée est inférieure à la valeur de référence ; et
dans une situation où la valeur mesurée est inférieure à la valeur de référence, l'incrémentation (814) d'un compte représentant le nombre de valeurs mesurées basses.

6. Procédé selon l'une quelconque des revendications 1 à 5, le procédé comprenant en outre :
la détermination (702) d'un ensemble de valeurs mesurées anormales à partir du sous-ensemble de valeurs mesurées basses ; et
la génération (704) d'un sous-ensemble modifié de valeurs mesurées basses en supprimant l'ensemble de valeurs mesurées anormales du sous-ensemble de valeurs mesurées basses,
dans lequel l'ensemble de valeurs de référence mises à jour est déterminé sur la base du sous-ensemble modifié de valeurs mesurées basses.

7. Procédé selon l'une quelconque des revendications 1 à 6, dans lequel la détermination (404) de l'ensemble de valeurs de référence mises à jour comprend :
pour chaque intervalle de facteurs de référence dans l'ensemble d'intervalles de facteurs de référence :
la détermination (904) d'une valeur mesurée basse moyenne pour l'intervalle de facteurs de référence en calculant la moyenne du sous-ensemble de valeurs mesurées basses associées à l'intervalle de facteurs de référence ; et
le réglage (906) de la valeur de référence mise à jour pour l'intervalle de facteurs de référence à la valeur mesurée basse moyenne pour l'intervalle de facteurs de référence.

8. Procédé selon l'une quelconque des revendications 1 à 7, le procédé comprenant en outre :
la mesure (410) d'une valeur de données brutes associée à un intervalle de facteurs de référence opérationnels actuel ; et
la génération (412) d'une valeur de données corrigée sur la base de :
la valeur de données brutes, et
une valeur de référence mise à jour associée à l'intervalle de facteurs de référence opérationnels actuel.

9. Procédé selon l'une quelconque des revendications 1 à 8, dans lequel l'ensemble de valeurs mesurées comprend des valeurs mesurées historiques reçues d'un serveur central ou d'une pluralité de dispositifs connectés.

10. Procédé selon l'une quelconque des revendications 1 à 9, dans lequel le procédé est lancé après avoir attendu un intervalle de temps prédéterminé.

11. Procédé selon l'une quelconque des revendications 1 à 10, dans lequel le procédé est lancé après l'occurrence d'un événement prédéterminé.

12. Procédé selon l'une quelconque des revendications 1 à 11, dans lequel le procédé comprend en outre le stockage d'un tableau de facteurs de référence comprenant chaque valeur de référence pour chaque intervalle de facteurs de référence.

13. Appareil (200) comprenant au moins un processeur (202) et au moins une mémoire (204) incluant un code de programme informatique, l'au moins une mémoire (204) et le code de programme informatique étant configurés pour amener, grâce à l'au moins un processeur (202), l'appareil (200) à exécuter l'un des procédés selon l'une quelconque des revendications 1 à 12.

14. Produit de programme informatique comprenant au moins un support non transitoire lisible par ordinateur présentant des instructions de programme lisibles par ordinateur stockées dans celui-ci, les instructions de programme lisibles par ordinateur comprenant des instructions qui, lorsqu'elles sont exécutées par un appareil (200), sont configurées pour amener l'appareil (200) à exécuter au moins un des procédés selon l'une quelconque des revendications 1 à 12.
